# EUROPEAN PATENT APPLICATION

(11) **EP 4 684 808 A1**
(43) Date of publication of application: **28.01.2026**
(21) Application number: 24190114.9
(22) Date of filing: 22.07.2024
(51) Int. Cl.: A61K 48/00, C12N 15/86

(54) **PLASMIDS, TRANSGENES, VECTORS AND MEDICAL USES COMPRISING ADAMTS13**

(71) Applicant: Evotec International GmbH, 22419 Hamburg (DE)
(72) Inventor: ROTTENSTEINER, Hanspeter, Dr., 2304 Orth an der Donau (AT); FEICHTINGER, Georg, Dr., 2304 Orth an der Donau (AT); PLAIMAUER, Barbara, Dr., 2304 Orth an der Donau (AT); HÖLLRIEGL, Werner, 2304 Orth an der Donau (AT); GLANTSCHNIG, Helmut, Dr., 2304 Orth an der Donau (AT)
(74) Representative: Maiwald GmbH

(57) **Abstract**

The present invention provides gene therapy plasmids and transgenes comprising a variant of ADAMTS13, recombinant virus vectors for delivery of said viral plasmids and transgenes and medical uses relating to the treatment of ADAMTS13 insufficiency.

## Description

### FIELD OF THE INVENTION

The present invention provides gene therapy plasmids and transgenes comprising a variant of ADAMTS13, recombinant virus vectors for delivery of said viral plasmids and transgenes and medical uses relating to the treatment of ADAMTS13 insufficiency.

### Background of the invention

Thrombotic Thrombocytopenic Purpura (TTP) is characterized by periodic episodes of tissue ischemia due to extensive microvascular thrombosis. TTP is defined by a mechanical hemolytic anemia, severe thrombocytopenia and systemic visceral ischemia causing multi-organ failure in the absence of urgent frontline therapeutic plasma exchange.

Patients with TTP lack ADAMTS13 (a disintegrin and metalloprotease with thrombospondin type 1 motif, member 13) activity (<10% of normal activity), a protease that cleaves the ultra-large multimers of von Willebrand factor (UL-VWF) that contribute to the formation of these thrombi. Current therapy consists of regular plasma infusions to replace the deficient or defective ADAMTS13. So far, severe ADAMTS13 deficiency is the only highly sensitive and specific marker for TTP (Alwan F et al. 2019, Blood 133:1644-1651; Scully M et al. 2017, J Thrombosis Haemostasis 12:312-322.)

In most cases, ADAMTS13 deficiency is acquired (immune-mediated TTP, iTTP) via specific auto-antibodies, increasing ADAMTS13 clearance and/or inhibiting its catalytic activity.

Congenital TTP (cTTP) represents 10% of all TTP cases and results from loss-of-function mutations of ADAMTS13 causing a reduced proteolytic activity on UL-VWF of <10% of the physiological levels. Despite the genetic predisposition to the disease, symptoms may not be apparent at very young age as additional predisposing factors (e.g. sex) and precipitating factors (e.g. acute infection, pregnancy) typically result in acute TTP episodes in cTTP patients.

Once diagnosed, cTTP patients remain at increased risk of death (10-20%) despite receiving standard of care prophylactic as well as acute treatments, as required. Note that relative insufficiency of ADAMTS13 also may play a role in other hypercoagulative disease states (e.g. sickle-cell disease Tsai HM 2004, J Thrombosis Haemostasis 2:1510-1514.).

ADAMTS13 is physiologically released into the circulation by stellate cells in the liver and, to a lesser degree by endothelial cells. Interaction of ADAMTS13 with VWF and proteolytic cleavage is regulated by shear-induced unfolding of VWF, causing the presentation of VWF domains A1-A3. The stabilizing interaction between functional domains from ADAMTS13 with corresponding sequences within domain A2 of VWF allows initiation of the proteolytic activity (Muia et al. 2014, Proc Nat Acad Sci 111:18584-18589).

An enzyme replacement therapy (ERT) providing recombinant human ADAMTS13 has completed a Phase-3 clinical trial and has been approved for the treatment of cTTP by FDA. Viral gene therapy using *Dependoparvovirus* such as AAV could provide a suitable alternative to treatment with plasma derived or recombinant enzyme replacement therapy.

*Dependoparvovirus* are a genus of viruses composed of a single-stranded DNA genome encapsidated in a 25 nm non-enveloped capsid. Gene therapy vectors based on *Dependoparvovirus,* such as adeno-associated viruses (AAV), have proven safe and efficacious in numerous clinical trials (Kuzmin et al. 2021, Nature Reviews Drug Discovery 20:173-174).

However, the large size of the gene encoding for ADAMTS13 (about 4.3 kilobases) makes it a poor candidate for AAV-mediated gene therapy, as the maximum packaging capacity of AAV is about 4.5-4.8 kilobases, including coding sequence and regulatory elements.

Current experimental approaches use a truncated version of ADAMTS13, the MDTCS variant. However, this variant lacks the regulatory protein domain responsible for the fine-tuned modulation of the proteolytic activity (Jin et al. 2013, Blood 121,19:3825-3853).

Thus, there is a need for gene therapeutic approaches that take into account the packaging capacity of AAV and at the same time retain the critical functional and regulatory domains of ADAMTS13.

### Objectives and Summary of the invention

The present invention solves the above-mentioned problem by providing a gene therapy plasmid suitable for viral delivery encoding variants of ADAMTS13 (a disintegrin and metalloprotease with thrombospondin type 1 motif, member 13) that are smaller than 4.3 kilobases (full length ADAMTS13) in length and comprise domains that allow for autoregulation of ADAMTS13 while retaining full functionality. The gene therapy plasmid further comprises regulatory elements for effective *in vivo* expression. The inventors were able to show that treatment with the gene therapy plasmid according to the invention reduced clinical signs of acute TTP in ADAMTS13^{-/-}knock-out mice.

Hence, in a first aspect, the invention provides a gene therapy plasmid comprising a recombinant gene expression cassette, comprising a nucleic acid sequence encoding a variant of ADAMTS13, wherein the nucleic acid sequence encoding a variant of ADAMTS13 comprises one proximal thrombospondin domain and at least one distal thrombospondin domain of ADAMTS13; wherein the gene therapy plasmid is suitable for viral delivery.

Wild-type full length human ADAMTS13 comprises a short propeptide, the proximal domains MDTCS (metalloprotease (M), disintegrin-like (D), thrombospondin-1 (T1), Cys-rich (C), and spacer (S) domains) followed by the distal domains thrombospondin-2 (T2), thrombospondin-3 (T3), thrombospondin-4 (T4), thrombospondin-5 (T5), thrombospondin-6 (T6), thrombospondin-7 (T7) and thrombospondin-8 (T8) and 2 CUB (complement components C1r and C1s, sea urchin protein Uegf, and bone morphogenetic protein-1) domains CUB1 and CUB2. Rodent ADAMTS13 has an additional distal thrombospondin-6a (T6a) domain. Variants of ADAMTS13 may have deletions of one or more domains, while specific domains remain intact. In one embodiment, ADAMTS13 is human ADAMTS13.

In one embodiment, the nucleic acid sequence encoding a variant of ADAMTS13 comprises a nucleic acid sequence encoding thrombospondin-1 (T1) domain and a nucleic acid sequence encoding thrombospondin-8 (T8) domain of ADAMTS13.

In one embodiment, the nucleic acid sequence encoding a variant of ADAMTS13 comprises one proximal thrombospondin domain and at least two distal thrombospondin domains of ADAMTS13.

In one embodiment, the nucleic acid sequence encoding a variant of ADAMTS13 further comprises a nucleic acid sequence encoding thrombospondin-7 (T7) domain of ADAMTS13. Hence, in this specific embodiment, the nucleic acid sequence encoding a variant of ADAMTS13 comprises a nucleic acid sequence encoding thrombospondin-1 (T1) domain, a nucleic acid sequence encoding thrombospondin-7 (T7) domain and a nucleic acid sequence encoding thrombospondin-8 (T8) domain of ADAMTS13.

In one embodiment, the nucleic acid sequence encoding a variant of ADAMTS13 comprises one proximal thrombospondin domain and at least three distal thrombospondin domains of ADAMTS13.

In one embodiment, the nucleic acid sequence encoding a variant of ADAMTS13 further comprises a nucleic acid sequence encoding thrombospondin-2 (T2) domain of ADAMTS13. Hence, in this specific embodiment, the nucleic acid sequence encoding a variant of ADAMTS13 comprises a nucleic acid sequence encoding thrombospondin-1 (T1) domain, a nucleic acid sequence encoding thrombospondin-2 (T2) domain, a nucleic acid sequence encoding thrombospondin-7 (T7) domain and a nucleic acid sequence encoding thrombospondin-8 (T8) domain of ADAMTS13.

In another embodiment, the nucleic acid sequence encoding a variant of ADAMTS13 further comprises a nucleic acid sequence encoding at least one CUB (complement components C1r and C1s, sea urchin protein Uegf, and bone morphogenetic protein-1) domain of ADAMTS13. The at least one CUB domain is selected from the group consisting of CUB1 domain or CUB2 domain. In one embodiment, the nucleic acid sequence encoding a variant of ADAMTS13 further comprises a nucleic acid sequence encoding CUB1 domain and CUB2 domain of ADAMTS13.

In another embodiment, the nucleic acid sequence encoding a variant of ADAMTS13 further comprises a nucleic acid sequence encoding metalloprotease (M), disintegrin-like (D), Cys-rich (C) and spacer (S) domains of ADAMTS13. Hence, in one particular embodiment, the nucleic acid sequence encoding a variant of ADAMTS13 comprises nucleic acid sequences encoding metalloprotease (M) domain, disintegrin-like (D) domain, Cys-rich (C) domain, spacer (S) domain, thrombospondin-1 (T1) domain, thrombospondin-2 (T2) domain, thrombospondin-7 (T7) domain, thrombospondin-8 (T8), CUB1 domain and CUB2 domain of ADAMTS13.

Preferably, the order of domains from N-terminus to C-terminus is metalloprotease (M) domain, disintegrin-like (D) domain, thrombospondin-1 (T1), Cys-rich (C) domain, spacer (S) domain, thrombospondin-2 (T2) domain, thrombospondin-7 (T7) domain, thrombospondin-8 (T8), CUB1 domain and CUB2 domain of ADAMTS13. The M domain may further be preceded by a nucleic acid sequence encoding the propeptide domain of ADAMTS13.

In one particular embodiment, the nucleic acid sequence encoding a variant of ADAMTS13 encodes an ADAMTS13 variant with deletion (del) of thrombospondin-3 (T3), -4 (T4), -5 (T5), and -6 (T6) domains (designated as deIT3-T6 or deIT3-6 or deltaT3-6 or ΔT3-6). Hence, this variant of ADAMTS13 comprises the domains from N-terminus to C-terminus: metalloprotease (M) domain, disintegrin-like (D) domain, thrombospondin-1 (T1), Cys-rich (C) domain, spacer (S) domain, thrombospondin-2 (T2) domain, thrombospondin-7 (T7) domain, thrombospondin-8 (T8), CUB1 domain and CUB2 domain of ADAMTS13, optionally preceded by the propeptide domain of ADAMTS13. In one embodiment, the ADAMTS13 deIT3-T6 variant does not comprise any other additional domains of ADAMTS13.

Hence, in one embodiment, the nucleic acid sequence encoding a variant of ADAMTS13 encodes a ADAMTS13 variant with deletion of thrombospondin-3, -4, -5, and -6 domains (delT3-T6), consisting of the domains from N-terminus to C-terminus: metalloprotease (M) domain, disintegrin-like (D) domain, thrombospondin-1 (T1), Cys-rich (C) domain, spacer (S) domain, thrombospondin-2 (T2) domain, thrombospondin-7 (T7) domain, thrombospondin-8 (T8), CUB1 domain and CUB2 domain of ADAMTS13, optionally preceded by the propeptide domain of ADAMTS13. In one embodiment, the amino acid sequence of the deIT3-T6 variant of ADAMTS13 is identical to wild-type ADAMTS13 wherein the thrombospondin-3, -4, -5, and -6 domains have been deleted.

A gene therapy plasmid is suitable for viral delivery if it can be packaged into a virus in place of its genome. For example, the genome of *Dependoparvovirus* is flanked by inverted terminal repeats (ITRs), and the nucleic acid sequence between ITRs is packaged into the virus during assembly and forms its genome. Hence, in one embodiment, the gene therapy plasmid comprises inverted terminal repeats. In a particular embodiment, the recombinant gene expression cassette is flanked by inverted terminal repeats.

Hence, in one embodiment, the recombinant gene expression cassette is flanked by viral ITR sequences and/or comprises a viral origin of replication (ORI).

The recombinant gene expression cassette also comprises a regulatory element. In one embodiment, the recombinant gene expression cassette further comprises a tissue-specific promoter operatively linked to the nucleic acid sequence encoding a variant of ADAMTS13.

In one particular embodiment, the tissue-specific promoter is a liver-specific promoter, preferably the liver-specific promoter is selected from the group consisting of HLP, hAAT, Alb, HBV, HCB, LP1, LP1b, and TTR; more preferably the liver-specific promoter is selected from the group consisting of HLP and HCB.

In one embodiment, the nucleic acid sequence encoding a variant of ADAMTS13 comprises a nucleic acid sequence that is at least 70% identical to the nucleic acid sequence set forth in SEQ ID NO: 1.

In one embodiment, the nucleic acid sequence encoding a variant of ADAMTS13 comprises a nucleic acid sequence encoding an amino acid sequence that is at least 70% identical to the amino acid sequence set forth in SEQ ID NO: 2.

In another embodiment, the recombinant gene expression cassette comprises a nucleic acid sequence that is at least 70% identical to the nucleic acid sequence set forth in SEQ ID NO: 3.

In another embodiment, the recombinant gene expression cassette including flanking ITRs comprises a nucleic acid sequence that is at least 70% identical to the nucleic acid sequence set forth in SEQ ID NO: 4.

In one embodiment, the variant of ADAMTS13 comprises a nucleic acid sequence encoding at least one CUB domain and is capable of autoinhibition of the metalloprotease.

In one embodiment, the variant of ADAMTS13 can be allosterically activated by its substrate von Willebrand Factor (VWF).

In some embodiments, the recombinant gene expression cassette further comprises
i) a nucleic acid sequence comprising one or more 5' intron sequences; and/or
ii) a nucleic acid sequence comprising one or more Woodchuck Hepatitis Virus posttranscriptional regulatory element (WPRE) sequences.

In another aspect, the invention also provides an isolated polynucleotide comprising the gene therapy plasmid described herein.

In another aspect, the invention also provides an isolated polynucleotide comprising the recombinant gene expression cassette described herein.

In yet another aspect, the invention also provides a recombinant virus comprising the recombinant gene expression cassette described herein or the isolated polynucleotide described herein.

In one embodiment, the recombinant virus is from the genus *Dependoparvovirus,* preferably wherein the recombinant virus is an Adeno-associated virus (AAV).

In another aspect, the invention also provides a host cell comprising the gene therapy plasmid described herein, the isolated polynucleotide described herein, or the recombinant virus described herein.

In yet another aspect, the invention also provides a pharmaceutical composition comprising the gene therapy plasmid described herein, the isolated polynucleotide described herein, the recombinant virus described herein, or a host cell described herein, and one or more excipients.

In another aspect, the invention provides the gene therapy plasmid described herein, the isolated polynucleotide described herein, the recombinant virus described herein, the host cell described herein, or the pharmaceutical composition described herein for use as a medicament.

In one embodiment, the invention also relates to the use of the gene therapy plasmid described herein, the isolated polynucleotide described herein, the recombinant virus described herein, the host cell described herein, or the pharmaceutical composition described herein in the manufacture of a medicament.

In yet another embodiment, the invention provides a method of treatment, comprising administering the gene therapy plasmid described herein, the isolated polynucleotide described herein, the recombinant virus described herein, the host cell described herein, or the pharmaceutical composition described herein to a patient in need thereof.

In another aspect, the invention provides the gene therapy plasmid described herein, the isolated polynucleotide described herein, the recombinant virus described herein, the host cell described herein, or the pharmaceutical composition described herein for use in treatment of TTP. Preferably, the TTP is Congenital Thrombotic Thrombocytopenic Purpura (cTTP).

In one embodiment, the treatment restores plasma activity level of ADAMTS13 to at least 10% of normal activity, preferably to at least 50% of normal activity; when measured with an activity assay using a von Willebrand Factor Fluorescence-Quenching Substrate.

In another embodiment, the treatment restores plasma activity level of ADAMTS13 to 50% to 150% of normal activity; when measured with an activity assay using a von Willebrand Factor Fluorescence-Quenching Substrate.

In one embodiment, the restored plasma activity level of ADAMTS13 remains stable over at least 3 months.

In one particular embodiment, the recombinant virus is administered at a dose between 3 × 10¹² vg/kg to 9 ×10¹² vg/kg.

### Figure Legends

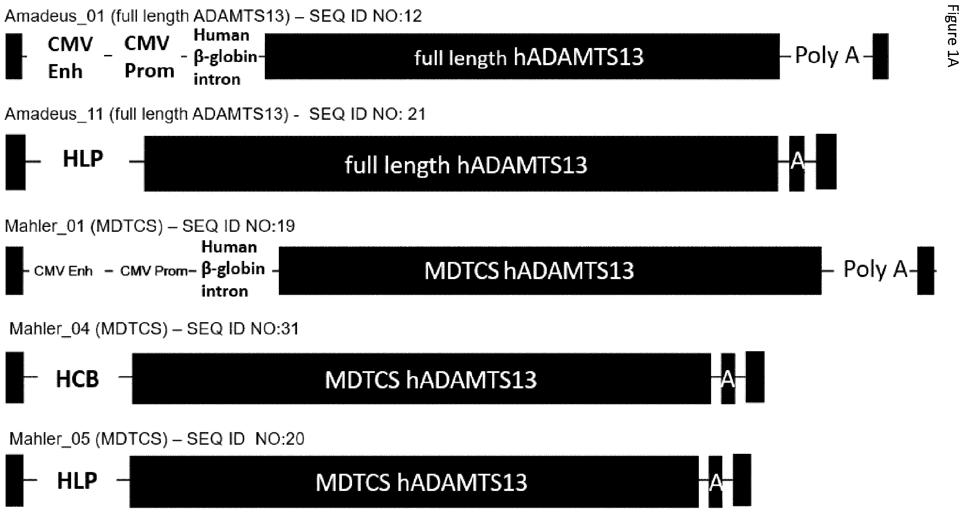

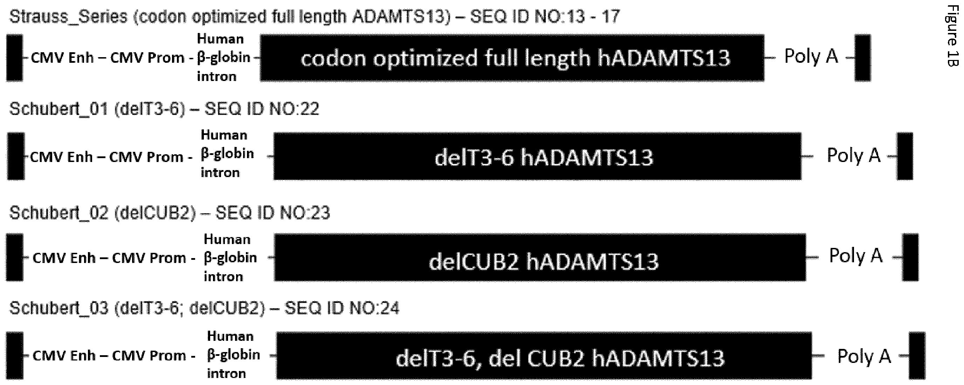

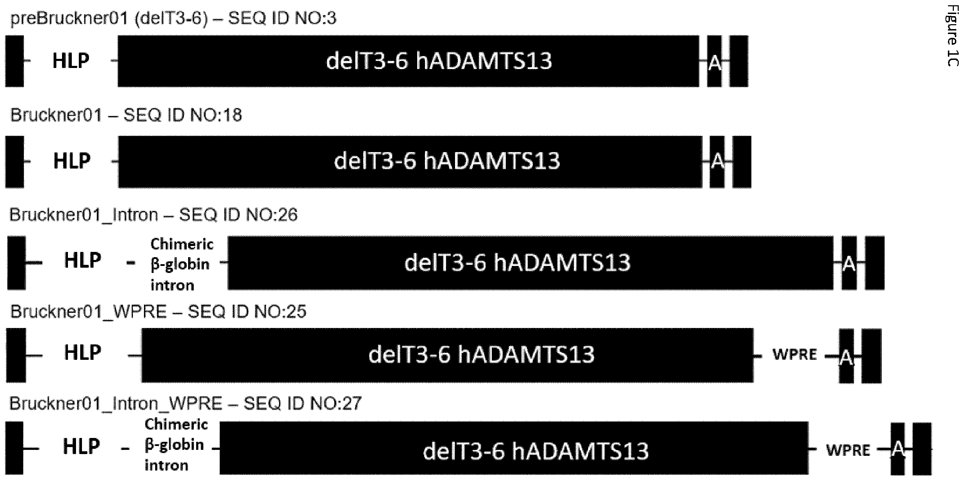

**Figure 1A****-C: Overview of selected vector expression cassettes used in Examples.**
   Not to scale. CMV: Cytomegalovirus, CMV Enh: CMV Enhancer (SEQ ID NO: 32), CMV Prom: CMV Promoter (SEQ ID NO: 33), HCB: synthetic promoter (SEQ ID NO: 37), HLP: hybrid liver promoter (SEQ ID NO: 36), human β-globin intron (SEQ ID NO: 40), chimeric β-globin intron (SEQ ID NO: 6), MDTCS: metalloprotease (M), disintegrin-like (D), thrombospondin-1 (T1), Cys-rich (C), and spacer (S) domains (SEQ ID NO: 11), deIT3-6: Thrombospondin domain 3-6 deletion (SEQ ID NO: 1), delCUB2: CUB2 domain deletion (SEQ ID NO: 41), deIT3-6 delCUB2 domain deletion (SEQ ID NO: 42), WPRE: Woodchuck Hepatitis Virus posttranscriptional regulatory element (SEQ ID NO: 7), PolyA (SEQ ID NO: 34), A: polyA short (SEQ ID NO: 35), elements flanking expression cassettes depict ITRs, all constructs in A and Band preBruckner01 of C comprise one set of ITRs (SEQ ID NO: 38, SEQ ID NO: 39) while all Bruckner01 constructs in C comprise another set of ITRs (SEQ ID NO: 8, SEQ ID NO: 9).

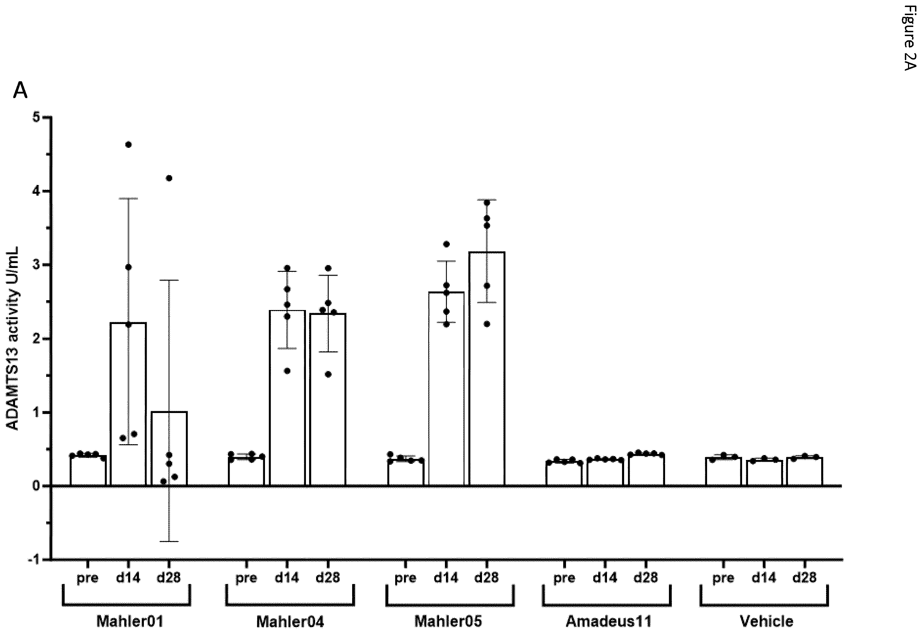

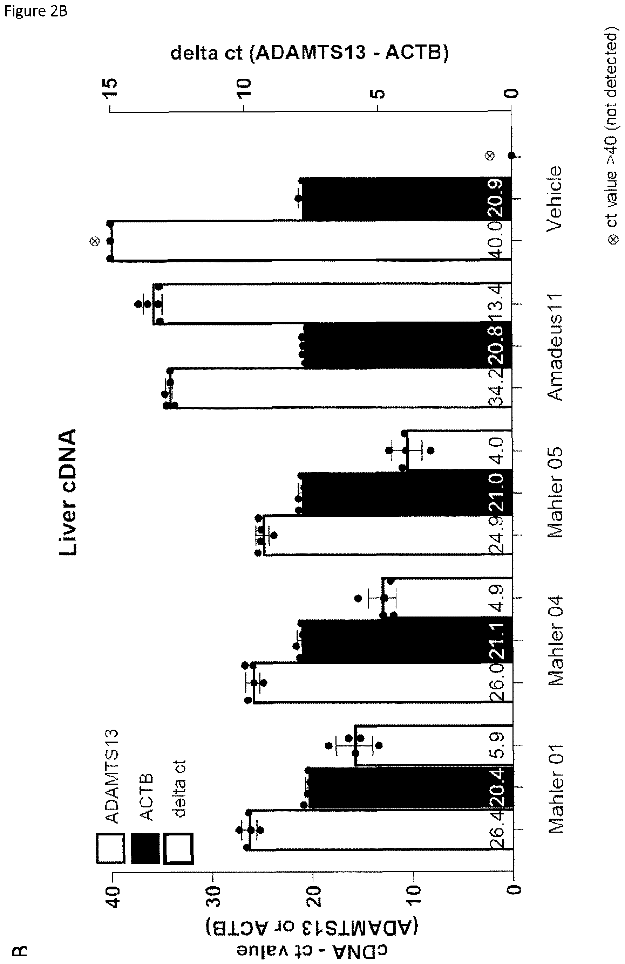

**Figure 2****: In vivo activity and expression levels of different ADAMTS13 variants.**
   **A:** ADAMTS13 activity was determined in plasma samples using the synthetic FRETS-VWF73 substrate (Fluorescence-Quenching Substrate based on 73 amino acids of the A2 domain of the von Willebrand Factor polypeptide, which contains the cleavage site for the VWF cleaving protease ADAMTS13). **B:** Total RNA from liver tissue (harvested at end of in-life phase, i.e. at day 28) was isolated, converted to cDNA by reverse transcription, and a duplex qPCR using target specific primers for cDNA expression of the ADAMTS13 transgene and the house keeping gene beta-actin (ACTB) was performed to measure cDNA expression of the ADAMTS13 transgene with ACTB as reference control. First bar shows ADAMTS13 CT values, second bar shows ACTB CT values, both depicted on the left y axis which indicates CT values. Third bar shows delta CT values (i.e. CT target ADAMTS13 minus CT ACTB), depicted on the right y axis. Schematic depiction of the design of the vector expression cassettes is depicted in Figure 1. Pre = pre-administration, d14, d28 = day 14 and day 28 after administration. Bars show mean and standard deviation. CT = cycle threshold.

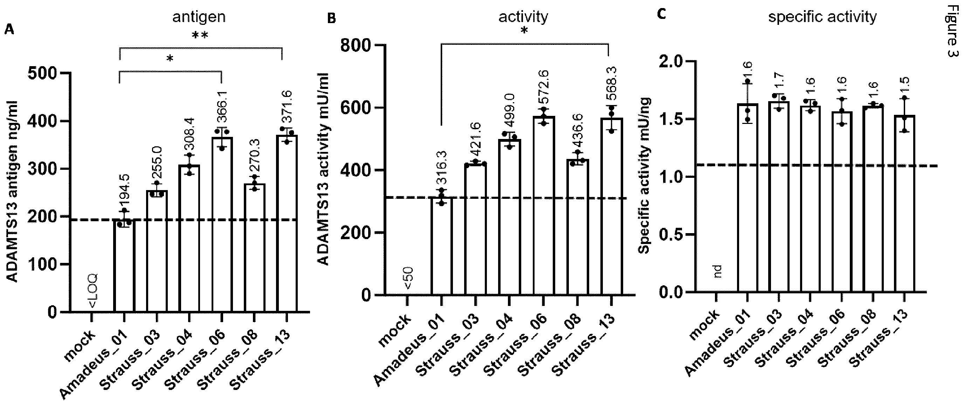

**Figure 3****: Codon-optimization of human full length ADAMTS13 for expression in human liver cells.**
   Human hepatoma HuH-7 cells were transfected with codon optimized DNA sequences encoding human full length ADAMTS13 in mammalian expression plasmids under the control of a constitutive promoter (CMV), and culture supernatants were collected after 72h of cultivation. **A:** Result of ELISA measuring ADAMTS13 antigen in culture supernatant (ng/ml). **B:** Result of FRETS-VWF73 activity assay measuring ADAMTS13 activity in culture supernatant (mU/ml), **C:** Specific activity, ADAMTS13 activity normalized to ADAMTS13 antigen levels (mU/ng). Bars show mean and standard deviation.

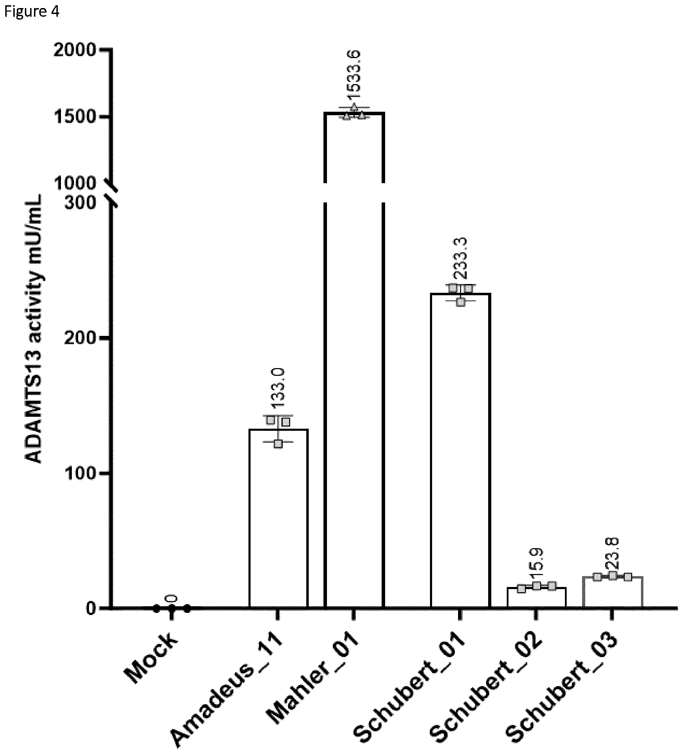

**Figure 4****: ADAMTS13 deletion variants.**
   ADAMTS13 activity (mU/ml) determined in culture supernatant of human hepatoma HepG2 cells transfected with expression plasmids for the indicated variants. Bars show mean and standard deviation. The variants depicted are deletion variants Schubert01, in which the thrombospondin domains 3, 4, 5, and 6 are deleted; Schubert02, in which the CUB2 domain is deleted; and Schubert03, in which both the thrombospondin domains 3, 4, 5, and 6 and the CUB2 domain are deleted, compared with Mahler01 (MDTCS variant) and Amadeus11 (full-length ADAMTS13).

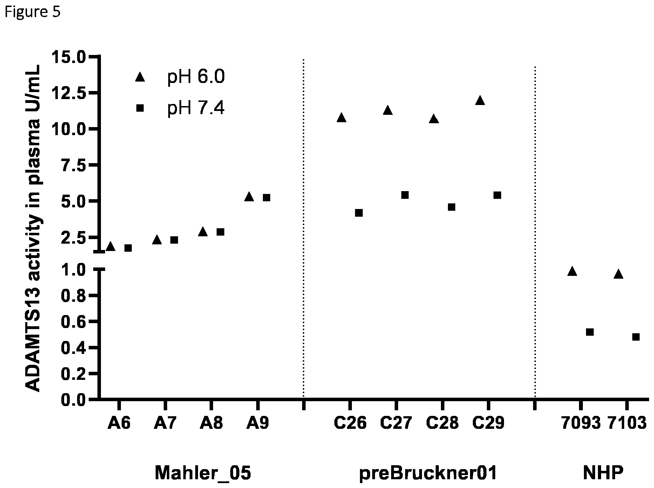

**Figure 5****: Auto-inhibition of ADAMTS13 enzymatic activity.**
   ADAMTS13 activity (U/ml, y axis) was measured in plasma samples from ADAMTS13 KO mice treated with the indicated constructs at assay conditions of pH 6.0 (triangles) and pH 7.4 (squares). X axis indicates the identification numbers of individual mice. NHP, normal human plasma as control, Mahler05 (MDTCS variant), preBruckner01 (deIT3-6 variant).

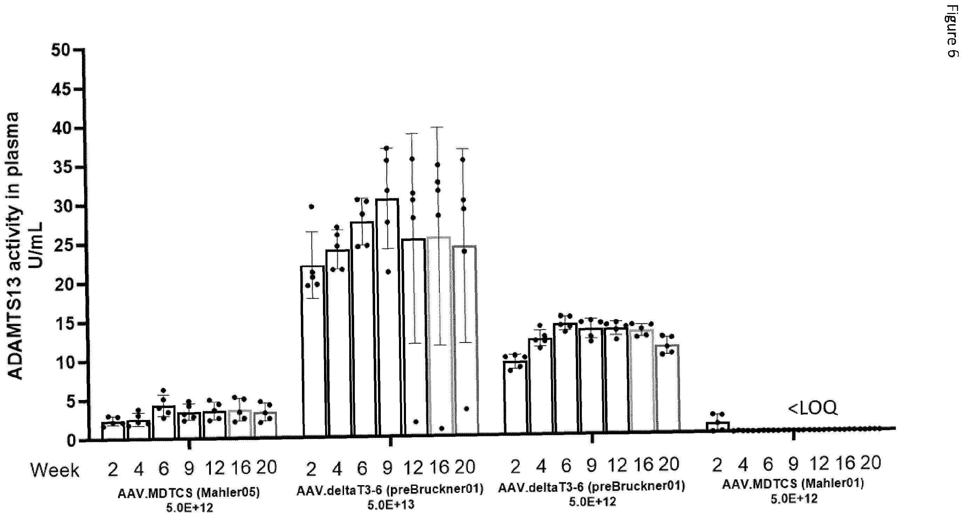

**Figure 6****: Liver directed expression of ADAMTS13 activity mediated by transduction with AAVdeIT3-6 (preBruckner01).**
   ADAMTS13 activity (U/ml, y axis) was measured in plasma samples from ADAMTS13 KO (A13 KO) mice treated with the indicated constructs at the indicated vector genome concentrations (vg/kg). Plasma samples were collected (retroorbital bleeds) 2, 4, 6, 9, 12, 16 and 20 weeks after dosing (x axis). ADAMTS13 activity was determined in plasma samples using FRETS-VWF73 substrate. Bars indicate the group mean +/- SD, N=5/group; Age range of A13 KO mice at dosing: 16 - 18 weeks; Age range at pharmacologic challenge: 22 - 24 weeks; Age range at study end week 24: 41 - 42 weeks. LOQ = limit of quantification.

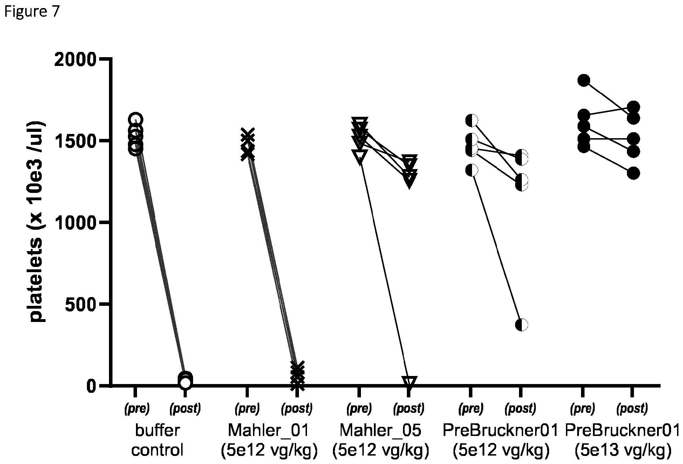

**Figure 7****: Disease-modification by liver specific lead vector in challenged ADAMTS13 KO mice.**
   Mice were administered with buffer (open circle), an irrelevant ineffective vector (Mahler01, cross), AAV-MDTCS (5 × 10¹² vg/kg, Mahler05); AAV-delT3-6 (5 × 10¹² vg/kg, PreBruckner01, semi-filled circle) or AAV-deIT3-6 (5 × 10¹³ vg/kg, PreBruckner01, filled circle). The number of platelets was determined 3-4 weeks after treatments (pre-challenge) and again 24 hours after challenge with recombinant VWF (post-challenge). Pharmacologic challenge: 4 weeks post transduction. Age range at dosing: 16 - 18 weeks; Age range at pharmacologic challenge: 22 - 24 weeks; Age range at study end week 24: 41 - 42 weeks.

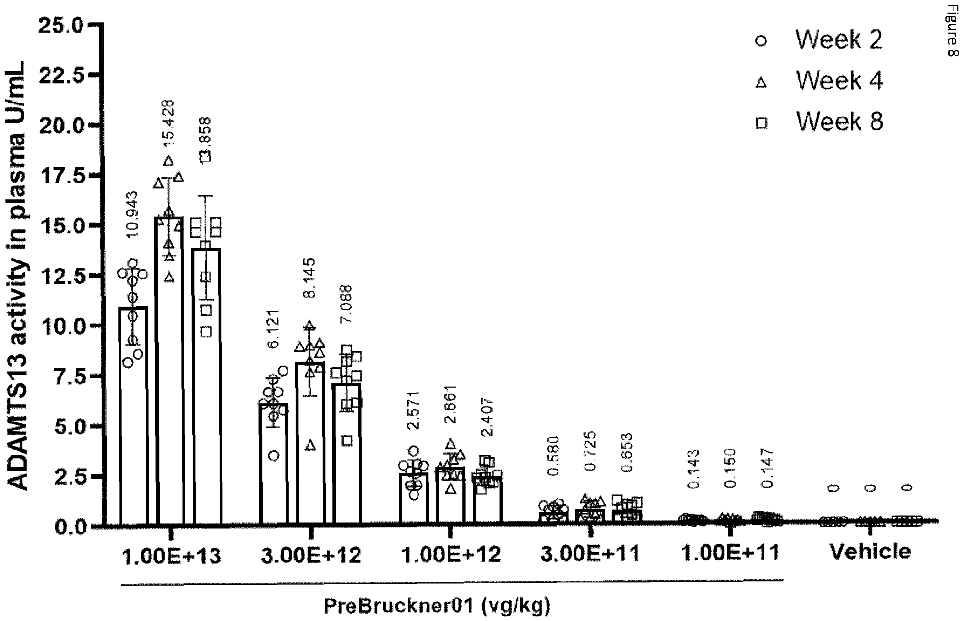

**Figure 8****: Dose finding study in ADAMTS13 KO mouse.**
   AAVdelT3-6 (PreBruckner01) was administered intravenously to male A13 KO mice at the indicated dose levels (vg/kg), and plasma samples were collected (retroorbital bleeds) 2, 4 and 8 weeks after dosing. ADAMTS13 activity was determined in plasma samples using FRETS-VWF73 substrate (U/ml, y axis). Phosphate-buffered-saline was administered in the control group (Vehicle).

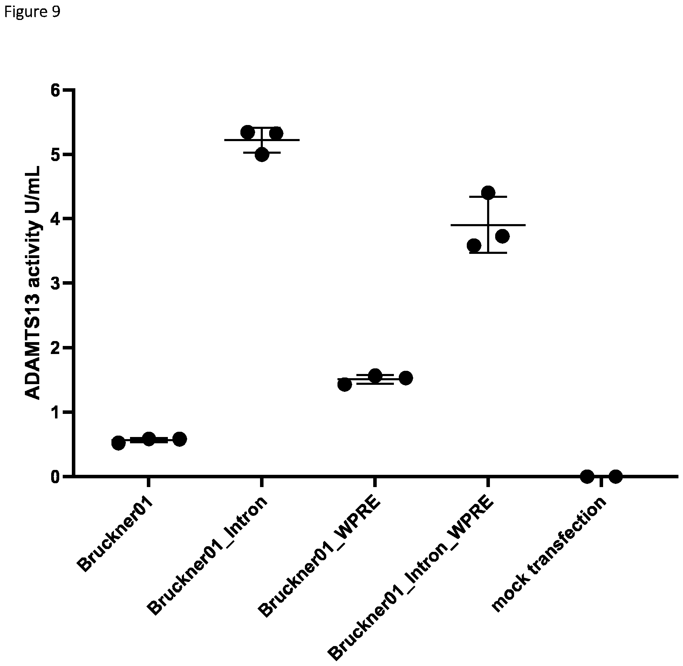

**Figure 9****: Expression cassette optimization.**
   Human liver cell line Huh-7 was transfected with expression cassettes encoding ADAMTS13 (A13) deIT3-6 under the control of a liver specific promoter (HLP_A13deIT3-6; Bruckner01); the HLP promoter and a beta-globin intron inserted in the 5` non-translated region (NTR) of the transgene (HLP_Intron A13deIT3-6, Bruckner01_lntron); the HLP promoter and Woodchuck Hepatitis Virus Posttranscriptional Regulatory Element inserted in the 3` NTR of the transgene (HLP_A13deIT3-6_WPRE, Bruckner01-WPRE); or the HLP promoter and a beta-globin intron inserted in the 5` NTR of the transgene and Woodchuck Hepatitis Virus Posttranscriptional Regulatory Element inserted in the 3` NTR of the transgene (HLP_Intron_A13delT3-6_WPRE, Bruckner01_lntron_WPRE). Cell culture supernatants were harvested and concentrated 9-fold and ADAMTS13 activity was determined using FRETS-VWF73 substrate (U/ml, y-axis).

### DETAILED DESCRIPTION OF THE INVENTION

### Definitions

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

As used herein, gene and protein names and symbols are all in capital letters regardless of species or molecule type for convenience. As such, the term "ADAMTS13" may refer to the RNA, DNA, cDNA or protein. If necessary, it is indicated in the text if the term refers to a DNA, RNA or protein.

The term "adeno-associated virus" or "AAV" as used herein refers to a virus of the family *Parvoviridae,* subfamily *Parvovirinae,* genus dependoparvovirus. "AAV" may be used to refer to the naturally occurring wild-type virus itself or derivatives thereof. The term covers all subtypes, serotypes and pseudotypes, and both naturally occurring and recombinant, synthetic or engineered variants. Furthermore, "AAV" refers to both the genetic components of the virus, e.g., the genome (positive or negative) and RNA transcripts thereof (either sense or antisense), proteins encoded by the genome (including structural and nonstructural proteins), and viral particles. The term "adeno-associated virus" (AAV), includes but is not limited to, AAV serotype 1, AAV serotype 2, AAV serotype 3 (including serotypes 3 A and 3B), AAV serotype 4, AAV serotype 5, AAV serotype 6, AAV serotype 7, AAV serotype 8, AAV serotype 9, AAV serotype 10, AAV serotype 11, AAV serotype 12, AAV serotype 13, AAVrh8, AAVrhIO, AAVrh.74, snake AAV, avian AAV, bovine AAV, canine AAV, equine AAV, ovine AAV, goat AAV, shrimp AAV, those AAV serotypes and clades disclosed by Issa et al. 2023, Cells 12(5):785 and Colón-Thillet et al. 2021, Virol J 18:85, and any other AAV occurring in nature (natural AAV isolate), or artificially designed (rational design, directed evolution) AAV capsid now known or later discovered.

One of the identifying characteristics of *Dependoparvovirus* is the encapsidation of a single-stranded DNA (ssDNA) genome that can be either the sense or anti-sense strand. In the case of AAV, the separate plus or minus polarity strands are packaged with equal frequency, and either is infectious. The small (about 4.8 kilobases) ssDNA genome consists of two open reading frames, Rep and Cap, flanked by two 145 base ITRs (inverted terminal repeats). Rep and Cap are translated to produce multiple distinct proteins (e.g., Rep78, Rep68, Rep52, and Rep40, required for the AAV life cycle; and VP1, VP2, and VP3, the capsid proteins). When constructing a nucleic acid to be delivered using AAV, the exogenous nucleic acid (e.g., an immunogenic polypeptide transgene) is placed between the two inverted terminal repeats (ITR), and Rep and Cap typically are supplied in trans. Being helper dependent, the adeno-associated viruses generally require a helper virus for a productive infection.

The term "inverted terminal repeat (ITR)" or "inverted terminal repeats (ITRs)" as used herein refers to nucleic acid sequences that are a component of a *Dependoparvovirus* transfer plasmid. The ITR regions are terminal repeats containing palindromic sequences that form hairpin-like structures. They usually occur in pairs of reverse-complement sequences and flank the genome of the virus on the 3' and 5' end. The ITR sequences contain the origin of replication and packaging signals. The nucleic acid sequence between the two ITR sequences, usually referred to as the genome or, in the case of a recombinant virus, the transgene, gets packaged into the virus. In the case of this particular invention, the gene expression cassette, which is flanked by ITRs, is packaged into the virus and comprises coding regions, signaling domains and regulatory regions. AAV virus have two ITR sequence of 145 bases each. The sequence of the ITR varies by virus species and, for example within species of AAV, by serotype. The terminal sequences of AAV are called homotelomeric termini, which refers to the occurance of two hairpins that are the same, i.e. perfectly reverse-complement sequences.

"Recombinant AAV" or "rAAV" refers to an engineered virus used as vector for *in vitro* or *in vivo* gene delivery. rAAVs are typically devoid of the *rep* gene and comprise an encapsidated genome which carries a therapeutic gene expression cassette in place of the genes necessary for virus production. The AAV genome is flanked by two ITRs at the ends that serve as the viral origins of replication and the packaging signal. In a rAAV vector, the only sequences of viral origin are the ITRs, which are needed to guide genome replication and packaging during vector production. The ITR-flanked rAAV genome can be cloned into plasmids and manipulated using standard molecular cloning techniques. Another version of rAAV is the self-complimentary AAV (scAAV), in which the coding region has been designed to form an intra-molecular double-stranded DNA template, thereby circumventing the need for second strand synthesis. The packaging capacity of a scAAV is about 2.4 kilobases, which is half of that of a typical rAAV (about 4.7-4.8 kilobases). Hence, the term recombinant when used for a virus also refers to a virus carrying modified or non-naturally occurring genetic material, such as a transgene or recombinant gene expression cassette, or genetic material from a different virus.

In order to produce an AAV virus, typically, three plasmids are needed. Firstly, a packaging plasmid which contains the structural and packaging genes, this includes nucleic acid sequences encoding capsid proteins. Secondly, an adenoviral helper plasmid which contains proteins needed for the virus to replicate, since AAV depends on proteins from other viruses, e.g. adenovirus, to replicate. Thirdly, a transfer plasmid, which contains the viral genome or transgene and carries the ITRs as a packaging signal. In the case of the present invention, the transfer plasmid is referred to as "viral plasmid" because it comprises the ITR sequences and the transgene, i.e. the recombinant gene expression cassette, for packaging. The ITR sequences and capsid protein genes can be from different species of *Dependoparvovirus* or from different serotypes of AAV. In some embodiments, the nucleic acid sequence encoding the ITRs and the nucleic acid sequence encoding the capsid proteins are from the same virus serotype. A "helper virus" for AAV refers to a virus that allows AAV (e.g., wild-type AAV) to be replicated and packaged by a mammalian cell. A variety of such helper viruses for AAV are known in the art, including adenoviruses, herpes viruses and poxviruses such as vaccinia. The adenoviruses encompass a number of different subgroups, although Adenovirus serotype 5 of subgroup C is most commonly used.

As used herein, the term "serotype" refers to a virus, such as AAV, which is identified by and distinguished from other viruses of the same genus based on capsid protein reactivity with defined antisera. For example, serotype AAV2 is used to refer to an AAV which contains capsid proteins encoded from the cap gene of AAV 2.

As used herein, the term "capsid protein" refers to a protein product of an open reading frame (ORF) encoded by the ssDNA genome of *Dependoparvovirus,* often termed "cap" ORF. The cap ORF encodes three capsid proteins Viral Protein 1 (VP1), VP2 and VP3, that are produced via differential splicing of the mRNA and use of alternate translational start codons.

"Packaging" as used herein refers to a series of subcellular events that results in the assembly and encapsidation of a cargo, e.g. a viral genome or recombinant viral genome or transgene. Thus, when a suitable polynucleotide is introduced into a packaging cell line under appropriate conditions, it can be assembled into a viral particle. The encapsidating protein shell is referred to as capsid. The capsid or shell has an inner and outer surface. The outer surface of the capsid is the part of the shell that is in contact with the environment.

The term "suitable for viral delivery" as used herein when referring to a plasmid means that the plasmid contains a nucleic acid sequence, such as a transgene or recombinant gene expression cassette, flanked by ITR sequences, which allow packaging into a virus. Preferably, the ITR sequences are from AAV2, and the transgene can be packaged into any *Dependoparovirus.* In one embodiment, the transgene is packaged into an AAV virus.

As used herein, "tropism" refers to the specificity of a capsid protein present in a virus or virus-like particle for infecting a particular type of cell or tissue. The tropism of a capsid for a particular type of cell or tissue may be determined by measuring the ability of a virus or virus-like particle comprising the capsid protein to infect or to transduce a particular type of cell or tissue, using standard assays that are well-known in the art.

The term "transduction" refers to a process for the introduction of an exogenous polynucleotide, e.g., a transgene in a viral vector, into a host cell leading to expression of the polynucleotide, e.g., the transgene, in the cell. Altered expression or persistence of a polynucleotide introduced via the virus can be determined by methods well known to the art including, but not limited to, protein expression, e.g., by ELISA, flow cytometry and Western blot, measurement of and DNA and RNA production by hybridization assays, e.g., Northern blots, Southern blots and gel shift mobility assays. Other methods used for the introduction of the exogenous polynucleotide include well-known techniques such as transfection, lipofection, viral infection, transformation, and electroporation, as well as non-viral gene delivery techniques. The introduced polynucleotide may be stably or transiently maintained in the host cell. A viral vector may also be referred to as "virion" or "viral particle".

The term "recombinant gene expression cassette" as used herein refers to a nucleic acid sequence that is part of a DNA plasmid and comprises a gene or transgene and regulatory sequences that is to be expressed by the cell into which the plasmid is introduced. The term "recombinant" denotes a gene expression cassette that is not naturally occurring but has been engineered and may comprise different elements that do not occur together in nature or additional synthetic sequences. In this particular example, the recombinant gene expression cassette encodes a transgene comprising the coding sequence of a gene, an Fc domain and a hydroxyapatite binding domain. The recombinant gene expression cassette may further comprise enhancers, polyA sequences, linkers and further functional sequences or domains. The recombinant gene expression cassette may also comprise recognition or binding motifs, such as miRNA binding sites.

The term "regulatory element" or "regulatory sequence" refers to non-coding nucleic acid sequences that control the transcription of genes or coding nucleic acid sequences. Regulatory elements include "enhancers" or "silencers", which are recognized and bound by transcription factors, leading to upregulation or downregulation of transcription, respectively. Regulatory elements also include "promoters", which are nucleic acid sequences that include a transcription initiation site and transcription factor binding sites. Promoters are typically located directly upstream of a coding region and are operably linked to said coding region, meaning that they initiate transcription of this coding region.

It is commonly recognized that the regulatory activity of promoters can differ depending on developmental timing and tissue specificity. In adult cells, tissues or organisms, the two main categories of promoters are tissue non-specific promoters and tissue-specific promoters.

"Tissue non-specific promoters" are also called "constitutive" or "ubiquitous" promoters, meaning that they are always active, independent from the cell type or tissue or developmental timing. It is however conceivable that they are specifically engineered to be repressed in an experimental setting. Furthermore, their strength of expression is not necessarily equal in all cell types, and it is conceivable that in some cases exhibit less or no expression in a specific minority of cell types.

"Tissue-specific promoters" or "regulated promoters" become active in the cell only in response to specific stimuli, for example specific transcription factors that are only present in one cell type or only in a small number of cell types. As used herein, the term "tissue-specific promoter" refers to a promoter that is predominantly active in a specific tissue in the adult organism. This does not exclude that the promoter is active, albeit to a lesser degree, in other cell types or tissues than the target tissue or in other developmental stages of the organism. Known tissue-specific promoters are functionally specific to the extent that they can be safely used for to target expression to a specific tissue *in vivo.* Such tissue-specific promoters are known, for example, for muscle tissue (e.g. desmin promoter, muscle creatine kinase (MCK) promoter), endothelial cells (e.g. angiopoietin-1 receptor (Tek/Tie2) promoter), neurons (e.g. synapsin 1 (Syn1) promoter), or liver (e.g. albumin (Alb) promoter, hybrid liver promoter (HLP), thyroxine binding globulin (TBG) promoter).

Promoters can also be synthetic or engineered, non-naturally occurring sequences. For example, promoters can be designed to achieve higher levels of expression or to be regulated by elements of a specific cellular pathway or other synthetic factors.

The term "polynucleotide" or "nucleic acid sequence" refers to a polymeric form of nucleotides of any length, including deoxyribonucleotides or ribonucleotides, or analogs thereof. A nucleic acid sequence is typically composed of a specific sequence of four nucleotide bases: adenine (A); cytosine (C); guanine (G); and thymine (T) (uracil (U) for thymine (T) when the nucleic acid sequence is RNA). Thus, the term polynucleotide sequence or nucleic acid sequence is the alphabetical representation of a polynucleotide molecule. A polynucleotide may comprise modified nucleotides, such as methylated or capped nucleotides and nucleotide analogs, and may be interrupted by non-nucleotide components. If present, modifications to the nucleotide structure may be imparted before or after assembly of the polymer. The term nucleic acid sequence, as used herein, refers interchangeably to double- and single-stranded polynucleotide molecules. Unless otherwise specified or required, any embodiment of the invention described herein that is a nucleic acid sequence encompasses both the double-stranded form and each of two complementary single-stranded forms known or predicted to make up the double-stranded form.

As used herein, "isolated" refers to a nucleic acid molecule or a nucleic acid sequence that has been substantially separated, produced apart from, or purified away from other biological components in the cell or tissue of an organism in which the component occurs, such as other cells, chromosomal and extrachromosomal DNA and RNA, and proteins. Nucleic acids and proteins that have been "isolated" include nucleic acids and proteins purified by standard purification methods. The term also embraces nucleic acids and proteins prepared by recombinant expression in a host cell as well as chemically synthesized nucleic acids and proteins. Isolated proteins or nucleic acids, or cells containing such, in some examples are at least 50% pure, such as at least 75%, at least 80%, at least 90%, at least 95%, at least 98%, or at least 100% pure.

"Host cells," "cell lines," "cell cultures," "packaging cell line" and other such terms denote higher eukaryotic cells, e.g., mammalian cells, such human cells, useful in the present invention. These cells can be used as recipients for recombinant vectors, viruses or other transfer polynucleotides, and include the progeny of the original cell that was transduced. It is understood that the progeny of a single cell may not necessarily be completely identical (in morphology or in genomic complement) to the original parent cell.

The terms "polypeptide" and "protein" are used interchangeably herein to refer to polymers of amino acids of any length. The terms also encompass an amino acid polymer that has been modified; for example, disulfide bond formation, glycosylation, acetylation, phosphorylation, lipidation, or conjugation with a labeling component. The term "amino acid sequence" as used herein likewise refers to a continuous string of amino acids. This term can be used interchangeably with the terms "polypeptide" or "protein". The term "amino acid sequence" may refer to a full-length protein, to a domain of a protein, to a functional region of a protein, or to a fragment or portion of a protein, wherein the fragment may be of any length.

"Sequence identity" or "percentage identity" and "sequence similarity" can be determined by alignment of two peptide or two nucleotide sequences using global or local alignment algorithms. Sequences may then be referred to as "substantially identical" or "essentially similar" when they are optimally aligned. For example, sequence similarity or identity can be determined by searching against databases such as FASTA, BLAST, etc., but hits should be retrieved and aligned pairwise to compare sequence identity. Two proteins or two protein domains, or two nucleic acid sequences can have "substantial sequence identity" if the percentage sequence identity is at least 70%, 75%, 80%, 85%, 90%, 95%, 98%, 99% or more, preferably 90%, 95%, 98%, 99% or more. Such sequences are also referred to as "variants" herein, e.g., other variants of a missing, deficient, and/or mutant protein or enzyme. I

Alternatively, the degree of sequence similarity between polynucleotides can be determined by hybridization of polynucleotides under conditions that form stable duplexes between homologous regions, followed by digestion with single-stranded-specific nuclease(s), and size determination of the digested fragments. Two DNA, or two polypeptide sequences are "substantially homologous" to each other when the sequences exhibit at least about 80-85%, preferably 85-90%, more preferably 90-95%, and most preferably 98-100% sequence identity to the reference sequence over a defined length of the molecules, as determined using the methods above. As used herein, substantially homologous also refers to sequences showing complete identity to the specified DNA or polypeptide sequence. DNA sequences that are substantially homologous can be identified in a Southern hybridization experiment under, for example, stringent conditions, as defined for that particular system. Defining appropriate hybridization conditions is within the skill of the art.

The term "composition" as used herein refers to a mixture comprising a therapeutically effective amount of the agent according to the present invention, i.e. a viral plasmid or recombinant virus of the invention, and one or more excipients. The term "excipient" as used herein may also be referred to as "pharmaceutically acceptable carrier", or "pharmaceutically acceptable excipient," "pharmaceutically acceptable diluent,", or "pharmaceutically acceptable vehicle," used interchangeably herein, refer to a non-toxic solid, semisolid or liquid filler, diluent, encapsulating material or formulation auxiliary of any conventional type.

A pharmaceutically acceptable carrier is essentially non-toxic to recipients at the dosages and concentrations employed and is compatible with other ingredients of the formulation. A pharmaceutically acceptable carrier will not inhibit otherwise adversely affect the function of the agent according to the present invention. Suitable carriers include, but are not limited to water, dextrose, glycerol, saline, ethanol, and any combination thereof. The carrier can contain additional agents such as wetting or emulsifying agents, pH buffering agents, or adjuvants, which enhance the effectiveness of the formulation.

As used herein, the term "excipient" refers to an inert substance which is commonly used as a diluent, vehicle, preservative, binder, or stabilizing agent, and includes, but is not limited to, proteins (e.g., serum albumin, etc.), amino acids (e.g., aspartic acid, glutamic acid, lysine, arginine, glycine, histidine, etc.), fatty acids and phospholipids (e.g., alkyl sulfonates, caprylate, etc.), surfactants (e.g., SDS, polysorbate, nonionic surfactant, etc.), saccharides (e.g., sucrose, maltose, trehalose, etc.) and polyols (e.g., mannitol, sorbitol, etc.).

The words "treat" or "treating" or "treatment" include palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In an aspect, the terms cover any treatment of a subject, including a mammal (e.g., a human), and includes: (i) preventing the undesired physiological change, disease, pathological condition, or disorder from occurring in a subject that can be predisposed to the disease but has not yet been diagnosed as having it; (ii) inhibiting the physiological change, disease, pathological condition, or disorder, i.e., arresting its development; or (iii) relieving the physiological change, disease, pathological condition, or disorder, i.e., causing regression of the disease.

For example, in an aspect, treating a disease or disorder can reduce the severity of an established a disease or disorder in a subject by 1 %-100% as compared to a control. In an aspect, treating can refer to a 1%, 2%, 3%, 4%, 5%, 6%, 7%, 8%, 9%, 10%, 20%, 30%, 40%, 50%, 60%, 70%, 80%, 90%, or 100% reduction in the severity of a disease or disorder (such as a genetic disease or disorder). For example, treating a disease or disorder can reduce one or more symptoms of a disease or disorder in a subject by 1 %-I 00% as compared to a control. In an aspect, treating can refer to 1%, 2%, 3%, 4%, 5%, 6%, 7%, 8%, 9%, 10%, 20%, 30%, 40%, 50%, 60%, 70%, 80%, 90%, 100% reduction of one or more symptoms of an established a disease or disorder. It is understood that treatment does not necessarily refer to a cure or complete ablation or eradication of a disease or disorder. However, in an aspect, treatment can refer to a cure or complete ablation or eradication of a disease or disorder.

An "individual" or "subject" treated in accordance with this invention refers to vertebrates, particularly members of a mammalian species, and includes but is not limited to domestic animals, sports animals, and primates, including humans. In one embodiment, the subject treated in accordance with this invention is a mammal. In one embodiment, the subject treated in accordance with this invention is a human. In another embodiment, the subject treated in accordance with this invention is a non-human mammal.

"Wild-type", "control" or "reference" gene expression, mRNA or protein levels are determined by a control sample, cell or organisms, or by averaging the expression levels from multiple control samples, cells or organisms. In the context of the present invention, the term "wild-type" or "control" refers to a cell or organism that is healthy or a sample from a subject that is healthy or to a cell or organism with a specific disease that is different from the disease to be treated.

The term "genetic disease" or "genetic disorder" as used herein refers to a disease caused by one or more mutations in a single gene (monogenic) or in multiple genes (polygenic). The genetic disease may be autosomal dominant, autosomal recessive, X-linked dominant, X-linked recessive, Y-linked or mitochondrial.

The term "gene therapy" as used herein refers to the alteration of endogenous gene expression by introduction of a therapeutic agent. Most commonly, gene therapy involves the introduction of foreign, i.e. heterologous, DNA or RNA into a cell or organism. This results in an increase or decrease of gene expression or in the replacement of a defective gene. Hence, gene therapy is not limited to monogenic diseases, but is suitable to treat any disease wherein the increase or decrease of gene expression can restore the levels of a gene that is aberrantly expressed. The introduction of a heterologous nucleic acid can be achieved with a vector, such as a *Dependoparovirus* vector comprising the viral gene therapy plasmid of the invention.

"Codon optimization" exploits the genetic code's redundancy, allowing for multiple codon options for a single amino acid. One of the goals is to enhance protein expression by specific target tissue/cells (e.g. liver) or increasing the effectiveness of therapy by lowering immunogenicity.

### Plasmid and Transgene

In a first aspect, the invention provides a gene therapy plasmid comprising a recombinant gene expression cassette, comprising a nucleic acid sequence encoding a variant of ADAMTS13, wherein the nucleic acid sequence encoding a variant of ADAMTS13 comprises one proximal thrombospondin domain and at least one distal thrombospondin domain of ADAMTS13; wherein the gene therapy plasmid is suitable for viral delivery.

In one embodiment, the nucleic acid sequence encoding a variant of ADAMTS13 comprises one proximal thrombospondin domain and at least two or at least three distal thrombospondin domains.

In a particular embodiment, the nucleic acid sequence encoding a variant of ADAMTS13 comprises one proximal thrombospondin domain and at least three distal thrombospondin domains, wherein the proximal thrombospondin domain is T1, and at least one of the three distal thrombospondin domains is T8.

In another embodiment, the nucleic acid sequence encoding a variant of ADAMTS13 further comprises a nucleic acid sequence encoding thrombospondin-7 (T7) domain of ADAMTS13. Hence, in this specific embodiment, the nucleic acid sequence encoding a variant of ADAMTS13 comprises a nucleic acid sequence encoding thrombospondin-1 (T1) domain, a nucleic acid sequence encoding thrombospondin-7 (T7) domain and a nucleic acid sequence encoding thrombospondin-8 (T8) domain of ADAMTS13.

In this particular embodiment, the nucleic acid sequence encoding a variant of ADAMTS13 comprises one proximal thrombospondin domain and at least three distal thrombospondin domains, wherein the proximal thrombospondin domain is T1, at least one of the three distal thrombospondin domains is T7, and at least one of the three distal thrombospondin domains is T8.

In one embodiment, the third distal thrombospondin domain may be selected from the group consisting of T2, T3, T4, T5, T6, T6a, T7 and T8. In a preferred embodiment, the third distal thrombospondin domain is T2.

In one embodiment, the three distal thrombospondin domains are three different thrombospondin domains. In another embodiment, at least two of the three distal thrombospondin domains are the same.

In yet another embodiment, the nucleic acid sequence encoding a variant of ADAMTS13 further comprises a nucleic acid sequence encoding thrombospondin-2 (T2) domain of ADAMTS13. Hence, in this specific embodiment, the nucleic acid sequence encoding a variant of ADAMTS13 comprises a nucleic acid sequence encoding thrombospondin-1 (T1) domain, a nucleic acid sequence encoding thrombospondin-2 (T2) domain, a nucleic acid sequence encoding thrombospondin-7 (T7) domain and a nucleic acid sequence encoding thrombospondin-8 (T8) domain of ADAMTS13.

Other nucleic acid sequences may be comprised between the nucleic acid sequences encoding thrombospondin domains. For example, other nucleic acid sequences may encode additional domains of ADAMTS13, linkers, regulatory regions, or selection markers.

In one embodiment, the variant of ADAMTS13 is a variant of human ADAMTS13. Human ADAMTS13 may be wild-type ADAMTS13 or a codon-optimized sequence variant.

In one particular embodiment, the nucleic acid sequence encoding a variant of ADAMTS13 encodes ADAMTS13 with deletion of thrombospondin-3, -4, -5, and -6 domains (deIT3-T6). Hence, this variant of ADAMTS13 comprises the domains from N-terminus to C-terminus: metalloprotease (M) domain, disintegrin-like (D) domain, thrombospondin-1 (T1), Cys-rich (C) domain, spacer (S) domain, thrombospondin-2 (T2) domain, thrombospondin-7 (T7) domain, thrombospondin-8 (T8), CUB1 domain and CUB2 domain of ADAMTS13, optionally preceded by the propeptide domain of ADAMTS13. In one embodiment, the ADAMTS13 deIT3-T6 variant does not comprise any other additional domains of ADAMTS13. This ADAMTS13 variant is exemplarily depicted in SEQ ID NO: 1 (nucleic acid sequence) and SEQ ID NO: 2 (amino acid sequence).

Hence, in one embodiment, the nucleic acid sequence encoding a variant of ADAMTS13 encodes a ADAMTS13 variant with deletion of thrombospondin-3, -4, -5, and -6 domains (delT3-T6), consisting of the domains from N-terminus to C-terminus: metalloprotease (M) domain, disintegrin-like (D) domain, thrombospondin-1 (T1), Cys-rich (C) domain, spacer (S) domain, thrombospondin-2 (T2) domain, thrombospondin-7 (T7) domain, thrombospondin-8 (T8), CUB1 domain and CUB2 domain of ADAMTS13, optionally preceded by the propeptide domain of ADAMTS13.

In one embodiment, the amino acid sequence of the deIT3-T6 variant of ADAMTS13 is identical to wild-type ADAMTS13 wherein the thrombospondin-3, -4, -5, and -6 domains have been deleted. In one embodiment, the nucleic acid sequence of the deIT3-T6 variant of ADAMTS13 is the wild-type nucleic acid sequence of human ADAMTS13, wherein T3-T6 have been deleted. In another embodiment, the nucleic acid sequence of the deIT3-T6 variant of ADAMTS13 is a codon-optimized nucleic acid sequence of human ADAMTS13. Wherein T3-T6 have been deleted. In another embodiment, the nucleic acid sequence of the deIT3-T6 variant of ADAMTS13 is a codon-optimized nucleic acid sequence of human ADAMTS13 for optimized liver expression, wherein T3-T6 have been deleted.

In one embodiment, the nucleic acid sequence encoding a variant of ADAMTS13 comprises a nucleic acid sequence that is at least 70% identical to the nucleic acid sequence set forth in SEQ ID NO: 1.

In one embodiment, the nucleic acid sequence encoding a variant of ADAMTS13 comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 1.

In another embodiment, the recombinant gene expression cassette comprises a nucleic acid sequence that is at least 70% identical to the nucleic acid sequence set forth in SEQ ID NO: 3.

In one embodiment, the recombinant gene expression cassette comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 3.

In another embodiment, the recombinant gene expression cassette flanked by ITRs comprises a nucleic acid sequence that is at least 70% identical to the nucleic acid sequence set forth in SEQ ID NO: 4.

In another embodiment, the recombinant gene expression cassette flanked by ITRs comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 4.

In another embodiment, the gene therapy plasmid comprises a nucleic acid sequence that is at least 70% identical to the nucleic acid sequence set forth in SEQ ID NO: 5.

In another embodiment, the gene therapy plasmid comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 5.

In another embodiment, the gene therapy plasmid comprises a nucleic acid sequence that is at least 70% identical to the nucleic acid sequence set forth in SEQ ID NO: 18.

In another embodiment, the gene therapy plasmid comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 18.

In another embodiment, the recombinant gene expression cassette flanked by ITRs comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 25.

In another embodiment, the recombinant gene expression cassette flanked by ITRs comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 26.

In another embodiment, the recombinant gene expression cassette flanked by ITRs comprises a nucleic acid sequence that is at least 75%, at least 80%, at least 85%, at least 90%, at least 95%, at least 97%, at least 98%, at least 99%, at least 99.5% or 100% identical to the nucleic acid sequence set forth in SEQ ID NO: 27.

In one embodiment, the invention provides a gene therapy plasmid comprising a recombinant gene expression cassette, comprising a nucleic acid sequence encoding an HLP promoter, a deIT3-T6 variant of ADAMTS13, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the gene recombinant expression cassette is flanked by ITRs, preferably ITRs from AAV2.

In one embodiment, the invention provides a gene therapy plasmid comprising a recombinant gene expression cassette, comprising a nucleic acid sequence encoding an HLP promoter, a nucleic acid encoding a deIT3-T6 variant of ADAMTS13, wherein the nucleic acid encodes an amino acid at least 80% identical to the amino acid set forth in SEQ ID NO: 2, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the nucleic acid encodes an amino acid at least 80% identical to the amino acid set forth in SEQ ID NO: 2, wherein the gene recombinant expression cassette is flanked by ITRs, preferably ITRs from AAV2.

In one embodiment, the invention provides a gene therapy plasmid comprising a recombinant gene expression cassette, comprising a nucleic acid sequence encoding an HLP promoter, a nucleic acid encoding a deIT3-T6 variant of ADAMTS13, wherein the nucleic acid encodes an amino acid at least 90% identical to the amino acid set forth in SEQ ID NO: 2, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the nucleic acid encodes an amino acid at least 90% identical to the amino acid set forth in SEQ ID NO: 2, wherein the gene recombinant expression cassette is flanked by ITRs, preferably ITRs from AAV2.

In one embodiment, the invention provides a gene therapy plasmid comprising a recombinant gene expression cassette, comprising a nucleic acid sequence encoding an HLP promoter, a nucleic acid encoding a deIT3-T6 variant of ADAMTS13, wherein the nucleic acid encodes an amino acid at least 95% identical to the amino acid set forth in SEQ ID NO: 2, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the nucleic acid encodes an amino acid at least 95% identical to the amino acid set forth in SEQ ID NO: 2, wherein the gene recombinant expression cassette is flanked by ITRs, preferably ITRs from AAV2.

In one embodiment, the invention provides a gene therapy plasmid comprising a recombinant gene expression cassette, comprising a nucleic acid sequence encoding an HLP promoter, a nucleic acid encoding a deIT3-T6 variant of ADAMTS13, wherein the nucleic acid encodes an amino acid at least 98% identical to the amino acid set forth in SEQ ID NO: 2, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the nucleic acid encodes an amino acid at least 98% identical to the amino acid set forth in SEQ ID NO: 2, wherein the gene recombinant expression cassette is flanked by ITRs, preferably ITRs from AAV2.

In one embodiment, the invention provides a gene therapy plasmid comprising a recombinant gene expression cassette, comprising a nucleic acid sequence encoding an HLP promoter, a nucleic acid encoding a deIT3-T6 variant of ADAMTS13, wherein the nucleic acid encodes an amino acid as set forth in SEQ ID NO: 2, wherein the gene therapy plasmid is suitable for viral delivery.

In one embodiment, the invention provides a gene therapy plasmid comprising a recombinant gene expression cassette, comprising a nucleic acid sequence encoding an HLP promoter, a deIT3-T6 variant of ADAMTS13, wherein the nucleic acid encodes an amino acid as set forth in SEQ ID NO: 2, wherein the gene recombinant expression cassette is flanked by ITRs, preferably ITRs from AAV2.

In one embodiment, the variant of ADAMTS13 comprises a nucleic acid sequence encoding at least one CUB domain and is capable of autoinhibition of the metalloprotease. For example, the variant deIT3-T6 comprises the CUB1 and CUB2 domains of ADAMTS13. In one embodiment, the activity of plasma ADAMTS13 expressed from the gene therapy plasmid of the invention is increased at pH6 compared to pH7.4. Autoinhibition of the metalloprotease domain occurs due to an intramolecular change. Hence, the catalytic activity of the metalloprotease domain is inhibited.

### Regulatory Element

The recombinant gene expression cassette also comprises a regulatory element. In one embodiment, the recombinant gene expression cassette comprises a tissue-specific promoter operatively linked to the nucleic acid sequence encoding a variant of ADAMTS13.

The tissue-specific promoter may be a naturally occurring promoter or a non-naturally occurring, engineered or synthetic promoter. In one embodiment, the tissue-specific promoter is an engineered promoter. In one embodiment, the tissue-specific promoter is a synthetic promoter. In another embodiment, the tissue-specific promoter is a hybrid promoter.

Conversely, in one embodiment, recombinant gene expression cassette does not comprise a tissue non-specific promoter.

A tissue non-specific promoter is a constitutively active promoter. Tissue non-specific promoters are usually derived from the regulatory regions of constitutively active genes, so-called housekeeping genes. Examples of tissue non-specific promoters include CAG (CMV early enhancer/chicken β-Actin), CBA (chicken β-Actin), CMV (Cytomegalovirus promoter), HBA (human β-Actin), UBC (Ubiquitin C promoter), EF1α (elongation factor 1α promoter), PGK (phosphoglycerate kinase promoter), or SV40 (simian virus 40 promoter). Hence, in one embodiment, the regulatory element of b) is not a promoter selected from the group consisting of CAG, CBA, CMV, HBA, UBC, EF1α, PGK and SV40. In a particular embodiment, the regulatory element of b) is not a promoter selected from the group consisting of CAG, CBA and CMV. Preferably, the regulatory element of b) is not CAG.

In one embodiment, the tissue specific promoter is a liver-specific promoter. Exemplary liver-specific promoters are HLP (hybrid liver-specific promoter, Sandig et al. 1996), Gene Ther 3(11):1002-1009), hAAT (human α-antitrypsin), Alb (Albumin), HBV (hepatotrophic hepatitis B virus), HCB (synthetic promoter, Brown et al. 2018, Mol Ther Methods Clin Dev 9:57-69), LP1 (liver-restricted mini-human factor IX expression cassette, Nathwani et al. 2006, Blood 107 (7):2653-2661), LP1b (liver-specific promoter LP1b was engineered by combining elements from the human apolipoprotein E/C-I gene locus control region (ApoE-HCR) and a modified human α1 antitrypsin promoter (hAAT), Agudelo et al. 2020, Genome Research 30(1):107-117), TTR (transthyretin promoter, Yan et al. 1990, EMBO J 9:869-878). Hence, in one embodiment, the liver-specific promoter is selected from the group consisting of HLP, hAAT, Alb, HBV, HCB, LP1, LP1b, and TTR. In a preferred embodiment, the liver-specific promoter is HLP or HCB.

In a particularly preferred embodiment, the liver-specific promoter is HLP. Hence, in one embodiment, the recombinant gene expression cassette comprises an HLP promoter operatively linked to the ADAMTS13 variant described above. In one embodiment, the recombinant gene expression cassette comprises the ADAMTS13 deIT3-T6 variant and an HLP promoter operatively linked to the ADAMTS13 variant.

The recombinant gene expression cassette may comprise additional regulatory elements such as introns such as a beta-globin intron (SEQ ID NO: 6) or Woodchuck Hepatitis Virus Posttranscriptional Regulatory Element (WPRE, SEQ ID NO: 7).

In one embodiment, the gene expression cassette comprises a beta-globin intron inserted 5' of the transgene. Hence, the beta-globin intron is inserted between the promoter and the coding sequence of the transgene. In another embodiment, the gene expression cassette comprises a WPRE inserted 3' of the transgene. In another embodiment, the gene expression cassette comprises a beta-globin intron inserted 5' of the transgene and a WPRE inserted 3' of the transgene. In a particular embodiment, the gene expression cassette comprises a beta-globin intron inserted 5' of the transgene and a WPRE inserted 3' of the transgene, wherein the transgene is ADAMTS13 deIT3-T6 variant, and wherein the plasma activity of the transgene product improves the overall activity level in ADAMTS13 deficient plasma to at least 10% of normal-plasma activity.

In another particular embodiment, the gene expression cassette comprises an HLP promoter followed by a beta-globin intron inserted 5' of the transgene and a WPRE inserted 3' of the transgene, wherein the transgene is ADAMTS13 deIT3-T6 variant, and wherein the plasma activity of the transgene product improves the overall activity level in ADAMTS13 deficient plasma to at least 10% of normal-plasma activity..

### ITRs

The ITRs can be from a wild-type virus variant, a non-naturally occurring virus variant, a synthetic virus variant or an engineered virus variant of a virus from the genus *Dependoparovirus.*

In a preferred embodiment, the ITRs are from a virus of the genus *Dependoparvovirus.* In an even more preferred embodiment, the ITRs are from an adeno-associated virus (AAV).

In one embodiment, the at least one ITR is from an AAV selected from the group consisting of AAV1, AAV2, AAV3, AAV4, AAV5, AAV6, AAV7, AAV8, AAV9, AAV10, AAV11, AAV12, AAV13. In another embodiment, the at least one ITR is from AAV2.

In one embodiment, the nucleic acid sequence of the 5' ITR is at least 80% identical to the nucleic acid sequence set forth in SEQ ID NO: 8 and the nucleic acid sequence of the 3' ITR is at least 80% identical to the nucleic acid sequence set forth in SEQ ID NO: 9.

In one embodiment, the nucleic acid sequence of the 5' ITR is at least 85%, at least 90%, at least 95%, at least 97%, at least 98%, at least 99%, at least 99.5% or 100% identical to the nucleic acid sequence set forth in SEQ ID NO: 8 and the nucleic acid sequence of the 3' ITR is at least 85%, at least 90%, at least 95%, at least 97%, at least 98%, at least 99%, at least 99.5% or 100%identical to the nucleic acid sequence set forth in SEQ ID NO: 9.

### Recombinant Virus

One aspect of the invention is the provision of a recombinant virus comprising the recombinant gene expression cassette described herein or the isolated polynucleotide described herein.

The recombinant virus is characterized by comprising a viral genome, i.e. a recombinant gene expression cassette, or transgene, flanked by ITRs, and a capsid. The capsid is composed of viral proteins (VP), including VP1, VP2 and VP3. The viral proteins are encoded by a *cap* gene. The virus species or serotype of the capsid refers to the virus species or serotype the cap gene, i.e. the viral proteins, originate from.

The capsid can be from a wild-type virus variant, a non-naturally occurring virus variant, a synthetic virus variant or an engineered virus variant of a virus from the genus *Dependoparovirus.*

In a preferred embodiment, the capsid is from a virus of the genus *Dependoparovirus.* In an even more preferred embodiment, capsid is from an adeno-associated virus (AAV).

Preferably, the capsid is from an AAV exhibiting liver tropism.

In one embodiment, the capsid is from a natural AAV serotype capable of transducing liver cells, or the capsid is an artificially designed AAV capsid capable of transducing liver cells. Examples of natural AAV serotypes capable of transducing liver cells are AAV8 and AAV9. Examples of artificially designed AAV capsids capable of transducing liver cells are AAV-LK03 (Lisowski et al. 2014, Nature 506:382-386) and AAV-NP40 (Paulk et al. 2018, Mol Ther 26:289-303).

Hence, in one embodiment, the capsid is selected from the group consisting of AAV8, AAV9, AAV-LK03 and AAV-NP40. In a preferred embodiment, the capsid is from AAV8.

Hence, in a particular embodiment, the capsid is selected from the group consisting of AAV8, AAV9, AAV-LK03 and AAV-NP40 and the recombinant gene expression cassette is flanked by two ITRs of AAV2. Hence, in a particular embodiment, the capsid is from AAV8 and the recombinant gene expression cassette is flanked by two ITRs of AAV2.

In another embodiment, the capsid is from AAV9 and the recombinant gene expression cassette is flanked by two ITRs of AAV2.

The invention also relates to a host cell comprising the viral plasmid, the isolated polynucleotide or the recombinant virus of the invention. A host cell can be any mammalian cell capable of being transduced with the viral plasmid and helper plasmids and producing the virus. The skilled person is familiar with suitable host cells, such as HEK293 cells or CHO cells.

### Medical Uses

In a further aspect, the invention also relates to a pharmaceutical composition comprising the gene therapy plasmid, the isolated polynucleotide, the recombinant virus or the host cell of the invention, and optionally one or more excipients.

Gene therapy can be conducted to enhance the level of expression of ADAMTS13. A recombinant virus comprising the recombinant gene expression cassette of the invention may be used to genetically alter cells either for ADAMTS13 marking, replacement of a missing or defective ADAMTS13, or insertion of a therapeutic ADAMTS13.

The introduction of a recombinant virus comprising the recombinant gene expression cassette of the invention may involve use of any number of delivery techniques (both surgical and non-surgical) which are available and well known in the art. Such delivery techniques, for example, include vascular catheterization, cannulization, injection, inhalation, endotracheal, subcutaneous, inunction, topical, oral, percutaneous, intra-arterial, intravenous, and/or intraperitoneal administrations.

In particular, for delivery of a virus comprising the viral plasmid of the invention to a tissue, any physical or biological method that will introduce the virus comprising the viral plasmid of the invention to a host cell or organism can be employed. In a preferred embodiment, the recombinant virus of the invention is administered intravenously.

Compositions of this invention may be used *in vivo* as well as *ex vivo. In vivo* gene therapy comprises administering the virus comprising the viral plasmid of the invention directly to a subject. Pharmaceutical compositions can be supplied as liquid solutions or suspensions, as emulsions, or as solid forms suitable for dissolution or suspension in liquid prior to use. For administration into the respiratory tract, one mode of administration is by aerosol, using a composition that provides either a solid or liquid aerosol when used with an appropriate aerosolubilizer device. Another mode of administration into the respiratory tract is using a flexible fiberoptic bronchoscope to instill the vectors. Typically, the viral vectors are in a pharmaceutically suitable pyrogen-free buffer such as Ringer's balanced salt solution (pH 7.4). Although not required, pharmaceutical compositions may optionally be supplied in unit dosage form suitable for administration of a precise amount.

The decision of whether to use *in vivo* or ex *vivo* therapy, and the selection of a particular composition, dose, and route of administration will depend on a number of different factors, including but not limited to features of the condition and the subject being treated. The assessment of such features and the design of an appropriate therapeutic or prophylactic regimen is ultimately the responsibility of the prescribing physician.

One objective of the invention is to provide an improved treatment for ADAMTS13 insufficiency.

Hence, in one aspect, the invention provides the gene therapy plasmid described herein, the isolated polynucleotide described herein, the recombinant virus described herein, the host cell described herein, or the pharmaceutical composition described herein for use in treatment of ADAMTS13 insufficiency.

Conditions that are characterized by ADAMTS13 insufficiency include Thrombotic Thrombocytopenic Purpura (TTP) and sickle cell disease.

Hence, the invention provides the gene therapy plasmid described herein, the isolated polynucleotide described herein, the recombinant virus described herein, the host cell described herein, or the pharmaceutical composition described herein for use in treatment of ADAMTS13 insufficiency, wherein the ADAMTS13 insufficiency is selected from the group consisting of TTP and sickle cell disease (SCD), as well as other diseases associated with an imbalance of VWF-ADAMTS13 ratios. Hence, the invention provides the gene therapy plasmid described herein, the isolated polynucleotide described herein, the recombinant virus described herein, the host cell described herein, or the pharmaceutical composition described herein for use in treatment of Thrombotic Thrombocytopenic Purpura (TTP). Preferably, the TTP is Congenital Thrombotic Thrombocytopenic Purpura (cTTP).

The invention also provides the gene therapy plasmid described herein, the isolated polynucleotide described herein, the recombinant virus described herein, the host cell described herein, or the pharmaceutical composition described herein for use in treatment of sickle cell disease.

In one embodiment, the treatment described herein prevents thrombocytopenia.

In one embodiment, the treatment described herein increases platelet count in a subject compared to an untreated subject.

In one embodiment, the gene therapy plasmid described herein, the isolated polynucleotide described herein, the recombinant virus described herein, the host cell described herein, or the pharmaceutical composition described herein for use according to the invention is administered to a subject who has previously received enzyme replacement therapy (ERT) for cTTP.

In one embodiment, the recombinant virus for use according to the invention is administered to a subject who has previously received at least 10 doses of enzyme replacement therapy for cTTP. In another embodiment, at least 20, at least 30, at least 40, at least 50, at least 60, at least 70, at least 80, at least 90, at least 100 doses of enzyme replacement therapy for cTTP.

In one embodiment, the subject's plasma ADAMTS13 activity level after single dose of gene therapy of the invention and discontinuation of ERT is within the therapeutic range of the plasma ADAMTS13 activity level during continued ERT without gene therapy.

The invention provides the gene therapy plasmid of the invention, the isolated polynucleotide of the invention, the recombinant virus of the invention, the host cell of the invention, or the pharmaceutical composition of the invention for use as a medicament.

The invention also provides the use of the gene therapy plasmid of the invention, the isolated polynucleotide of the invention, the recombinant virus of the invention, the host cell of the invention, or the pharmaceutical composition of the invention for the manufacture of a medicament.

The invention also provides a method of treatment comprising the administration of an effective amount of the gene therapy plasmid of the invention, the isolated polynucleotide of the invention, the recombinant virus of the invention, the host cell of the invention, or the pharmaceutical composition of the invention to a subject in need thereof.

Thus, the invention provides a method of treatment of Thrombotic Thrombocytopenic Purpura comprising the administration of an effective amount of the gene therapy plasmid of the invention to a subject in need thereof.

In one embodiment, the treatment is administered once. Particularly, in one embodiment, the treatment is administered once and the therapeutic effect remains stable over an extended period of time, preferably over the subject's lifetime. In one embodiment, the treatment is administered once and the therapeutic effect remains stable over at least 3 months. In one embodiment, the treatment is administered once and the therapeutic effect remains stable over at least 12 months.

In one embodiment, the treatment restores plasma activity level of ADAMTS13 to at least 10% of normal activity, when measured with an activity assay using a von Willebrand Factor Fluorescence-Quenching Substrate. In another embodiment, the treatment restores plasma activity level of ADAMTS13 to at least 20%, at least 30%, at least 40%, at least 50%, at least 60%, at least 70%, at least 80%, at least 90% or at least 100% of normal activity. Preferably, to at least 50% of normal activity.

In another embodiment, the treatment restores plasma activity level of ADAMTS13 to 50% to 150% of normal activity; when measured with an activity assay using a von Willebrand Factor Fluorescence-Quenching Substrate.

In mice, the preferred dose of recombinant virus is between 1 × 10¹² vg/kg to 8 × 10¹³ vg/kg. The dose of about 5 × 10¹² vg/kg is referred to as a low dose. The low dose may be between 1x 10¹² and 8 × 10¹² vg/kg in mice. The high dose may be between 1 × 10¹³ and 8 × 10¹³ vg/kg in mice. Particularly, the high dose may be 5 × 10¹³ vg/kg.

In one embodiment, the dose of recombinant AAV carrying a recombinant gene expression cassette comprising the ADAMTS13 deIT3-T6 variant is between 1 × 10¹² vg/kg to 8 × 10¹³ vg/kg. In one embodiment, administration of between 1 × 10¹² vg/kg to 8 × 10¹³ of recombinant AAV carrying a recombinant gene expression cassette comprising the ADAMTS13 deIT3-T6 variant restores ADAMTS13 activity to at least 50% of wild-type activity.

Several methods can be considered for prediction of a human dose (Zou 2023, BJCP 89(4):1393-1401), including prediction of AAV8-based and HLP promoter-based gene therapy and expression of secreted transgene (hFVlll, human Factor VIII) product by the human liver (i.e. rAAV8-HLPhFVIII-V3). In general, a factor 20x-30x can be applied in the scaling from mouse to human doses suggesting a reasonable, effective and feasible starting dose for a clinical trial. Hence, a extrapolated starting dose for a clinical trial in human subjects is estimated 2 × 10¹³ to 3 × 10¹³ vg/kg.

In one embodiment, the recombinant virus for use according to the invention is administered to a human subject in need thereof at a maximum dose of about 2 × 10¹² to about 2 × 10¹⁴ vg/kg (vector genomes per kg). In one embodiment, the recombinant virus for use according to the invention is administered to a human subject in need thereof at a maximum dose of about 2 × 10¹² to about 2 × 10¹⁴ vg/kg, wherein the recombinant virus comprises an AAV8 capsid. In one embodiment, the recombinant virus for use according to the invention is administered to a human subject in need thereof at a dose of 1 × 10¹³ to 3 × 10¹³ vg/kg (vector genomes per kg).

In particular, in mice, administration of a virus dose of 1 × 10¹¹ vg/kg was sufficient to restore about 10% of ADAMTS13 plasma activity, considering a wild-type plasma activity of 0.4 U/ml in mice (Figure 8). Administration of a virus dose of 3 × 10¹¹ vg/kg was sufficient to restore about 50% of ADAMTS13 plasma activity. Hence, using the formula above, a reasonable clinically feasible starting dose correcting ADAMTS13 levels to at least 10% of normal levels in human patients could include a dose of 2-3 × 10¹² vg/kg. A clinical dose correcting ADAMTS13 plasma activity levels to >50% of normal levels in humans could include 6-9 × 10¹² vg/kg. Thus, in one embodiment, the recombinant virus for use according to the invention is administered to a human subject in need thereof at a dose selected from the group consisting of 2 × 10¹² to 9 × 10¹² vg/kg, 3 × 10¹² to 9 × 10¹² vg/kg, 2 × 10¹² to 6 × 10¹² vg/kg, or 2 × 10¹² to 6 × 10¹² vg/kg.

The dose may need to be adjusted depending on the individual. Based on the above, higher doses may be used to reach ADAMTS13 plasma activity levels between 50%-150% of wild-type.

In one embodiment, the recombinant virus for use according to the invention is administered to a human subject in need thereof at a maximum dose of 2 × 10¹², 5 × 10¹², 1 × 10¹³, 2 × 10¹³ or 3 × 10¹³ vg/kg.

In one embodiment, the recombinant virus for use according to the invention is administered to a human subject in need thereof at a maximum dose of 1 × 10¹³, 1.5 × 10¹³, 2 × 10¹³, 2.5 × 10¹³, or 3 × 10¹³ vg/kg.

It is understood that in some circumstances, for example dose finding studies or clinical trials, the recombinant virus will be administered in higher doses. Hence, the recombinant virus for use according to the invention may be administered to a human subject in need thereof at a dose of 1 × 10¹⁴ or more.

### Non-viral gene therapy uses

It is understood that plasmids can be used in gene therapy without the need to vectorize i.e. to produce a virus (Liu-Chen S et al. 2018, Sci Rep. May 18;8(1):7859.). Plasmid DNA can be a component of non-viral gene therapy. For example, the gene therapy plasmid of the invention may be administered with lipid formulations compatible with gene therapy or lipid nanoparticles either ex-vivo or in-vivo. Such gene therapy lipids or lipid nanoparticles exhibit inherent liver tropism. Alternatively, plasmid DNA can be used as naked DNA and administered via Hydrodynamic Delivery.

### Examples

### Constructs used in the Examples

A schematic overview of constructs used in the subsequent Examples is shown in Figure 1. Table 1 shows an overview of all Sequences and their SEQ ID NOs.

The hADAMTS13 full-length construct comprises the full-length cDNA of human ADAMTS13 as provided in SEQ ID NO: 10.

The hADAMTS13 MDTCS construct comprises the MDTCS variant as described in Jin et al. and Zheng et al. (Jin et al. 2013, Blood 121, 19:3825-3829; Zheng et al. 2003, J Biol Chem. 278(32):30136-41), which is truncated after the spacer domain and is provided in SEQ ID NO: 11.

The hADAMTS13 deIT3-6 construct comprises a variant wherein the thrombospondin domains 3, 4, 5 and 6 are deleted. This variant is provided in SEQ ID NO: 1.

Mahler01 comprises an expression cassette consisting of CMV enhancer (CMV Enh, SEQ ID NO: 32) and CMV promoter (CMV Prom, SEQ ID NO: 33), followed by a human β-globin intron (SEQ ID NO: 40) and operatively linked to the MDTCS variant of ADAMTS13 (SEQ ID NO: 11), followed by PolyA (SEQ ID NO: 34), and flanked by AAV2 ITRs (SEQ ID NO: 38, SEQ ID NO: 39). Mahler01 expression cassette is depicted in SEQ ID NO: 19.

Mahler04 comprises an expression cassette consisting of a 146 bp HCB promoter (synthetic promoter, Brown et al. 2018, Mol Ther Methods Clin Dev 9:57-69, SEQ ID NO: 37), operatively linked to the MDTCS variant of ADAMTS13 (SEQ ID: 11), followed by PolyA (SEQ ID NO: 35), and flanked by ITRs (SEQ ID NO:38, SEQ ID NO:39) as depicted in SEQ ID NO: 31.

Mahler05 comprises an expression cassette consisting of a 252 bp HLP promoter (hybrid liver-specific promoter, Sandig et al. 1996, Gene Ther 3(11):1002-1009, SEQ ID NO: 36), operatively linked to the MDTCS variant of ADAMTS 13 (SEQ ID NO: 11), followed by PolyA (SEQ ID NO:35), and flanked by ITRs (SEQ ID NO:38, SEQ ID NO:39). Mahler05 expression cassette is depicted in SEQ ID NO: 20.

Amadeus11 comprises an expression cassette consisting of a 252 bp HLP promoter (SEQ ID NO: 36), operatively linked to full-length hADAMTS13 (SEQ ID NO: 10), followed by PolyA (SEQ ID NO: 35), and flanked by ITRs (SEQ ID NO: 38, SEQ ID NO: 39). Amadeus11 expression cassette is depicted in SEQ ID NO: 21.

Amadeus01 comprises an expression cassette consisting of CMV enhancer (CMV Enh, SEQ ID NO: 32) and CMV promoter (CMV Prom, SEQ ID NO: 33), followed by a human β-globin intron (SEQ ID NO: 40), operatively linked to full-length hADAMTS13 (SEQ ID NO: 10), followed by PolyA (SEQ ID NO: 34), and flanked by ITRs (SEQ ID NO: 38, SEQ ID NO: 39). Amadeus01 expression cassette is depicted in SEQ ID NO: 12.

PreBruckner01 comprises an expression cassette consisting of a 252 bp HLP promoter, (SEQ ID NO: 36) operatively linked to the hADAMTS13 deIT3-6 variant (SEQ ID NO: 1), followed by PolyA (SEQ ID NO: 35), flanked by ITRs (SEQ ID NO: 38, SEQ ID NO: 39) and is partially codon-optimized in a 48 nucleotide sequence segment. PreBruckner01 expression cassette is depicted in SEQ ID NO: 3. PreBruckner01 expression cassette plus ITRs is depicted in SEQ ID NO: 4.

Bruckner01 comprises an expression cassette (expression cassette SEQ ID NO: 18) consisting of a 252 bp HLP promoter (SEQ ID NO: 36), operatively linked to the hADAMTS13 deIT3-6 variant (SEQ ID NO: 1), followed by PolyA (SEQ ID NO: 35), and flanked by ITRs differing from PreBruckner01 ITR (Bruckner01 ITR sequences depicted in SEQ ID NO: 8 and SEQ ID NO: 9) and is partially codon-optimized in a 48 nucleotide sequence segment.

Bruckner01_Intron expression cassette (SEQ ID NO: 26) additionally comprises a 262 bp chimeric beta-globin intron (SEQ ID NO: 6). Bruckner01_WPRE expression cassette (SEQ ID NO: 25) additionally comprises a 248 bp Woodchuck Hepatitis Virus posttranscriptional regulatory element (SEQ ID NO: 7). Bruckner01_lntron_WPRE expression cassette (SEQ ID NO: 27) additionally comprises both a 262 bp chimeric beta-globin intron (SEQ ID NO: 6) and a 248 bp Woodchuck Hepatitis Virus posttranscriptional regulatory element (SEQ ID NO: 7).

Schubert01 (deIT3-6; 5147 bp), Schubert02 (delCUB2; 5552 bp) or Schubert03 (deIT3-6, delCUB2; 4757 bp) expression cassettes are depicted in SEQ ID NOs 22, 23 and 24 respectively.

Strauss (ST) 03, ST04, ST06, ST08 and ST13 carry an expression cassette with liver codon-optimized full length ADAMTS13. Sequences of ST03-ST13 are provided in SEQ ID NO: 13, 14, 15, 16 and 17.

**Table 1: Overview of all sequences described herein.**

| ITEM | SEQ ID NO: |
|---|---|
| hADAMTS13 deIT3-T6 cDNA | 1 |
| hADAMTS13 deIT3-T6 protein sequence | 2 |
| PreBruckner01 expression cassette | 3 |
| PreBruckner01 expression cassette and ITR | 4 |
| PreBruckner01 construct | 5 |
| chimeric chicken/rabbit beta-globin intron | 6 |
| WPRE | 7 |
| 5' ITR | 8 |
| 3' ITR | 9 |
| hADAMTS13 full length cDNA | 10 |
| hADAMTS13 MDTCS cDNA | 11 |
| Amadeus01 construct | 12 |
| Strauss03 construct | 13 |
| Strauss04 construct | 14 |
| Strauss06 construct | 15 |
| Strauss08 construct | 16 |
| Strauss13 construct | 17 |
| Bruckner01 expression cassette | 18 |
| Mahler01 construct CMV MDTCS | 19 |
| Mahler05 construct HLP MDTCS | 20 |
| Amadeus11 construct HLP full length | 21 |
| Schubert_01 | 22 |
| Schubert_02 | 23 |
| Schubert_03 | 24 |
| Bruckner 01_WPRE | 25 |
| Bruckner01_Intron | 26 |
| Bruckner01 Intron WPRE | 27 |
| hADAMTS13 forward primer | 28 |
| hADAMTS13 reverse primer | 29 |
| hADAMTS13 probe | 30 |
| Mahler_04 | 31 |
| CMV Enhancer | 32 |
| CMV Promoter | 33 |
| HGH PolyA | 34 |
| PolyA short (A) | 35 |
| HLP promoter | 36 |
| HCB promoter | 37 |
| PreBruckner 5' ITR | 38 |
| PreBruckner 3' ITR | 39 |
| Human beta globin intron | 40 |
| hADAMTS13 delCUB2: CUB2 domain deletion cDNA | 41 |
| hADAMTS13 delT3-6 delCUB2 domain deletion cDNA | 42 |

### Example 1: Experimental in-vivo evidence for the selection of liver specific promoter and deselection of full length ADAMTS13 as a payload for gene therapeutic vectors based on AAV

AAV8 based viral vectors carrying variants of ADAMTS13 and different promoters were tested for their ability to drive expression and provide sufficient plasma activity of ADAMTS13 in the liver.

AAV8 based vectors were administered intravenously to male wild-type C57/BL6 mice at the dose of 5 × 10¹² vg/kg (vector genomes per kilogram), and plasma samples were collected predose, and at 2 and 4 weeks after dosing (retroorbital bleeds). ADAMTS13 activity was determined in plasma samples using FRETS-VWF73 substrate (Fluorescence-Quenching Substrate based on the 73mer von Willebrand Factor polypeptide for the VWF cleaving protease ADAMTS13) (Kokame et al. 2005, BrJH 129 (1):93-100). Samples were measured against a reference standard of pooled normal human plasma with a regarded ADAMTS13 activity of 1U/mL, diluted to a working range from 0.08 to 0.005 U/mL. Liver tissue was analyzed by qPCR using target specific primers for RNA/cDNA expression of the ADAMTS13 transgenes (hADAMTS13 forward primer: TGGAGGAGATCCGCAT, SEQ ID NO: 28; hADAMTS13 reverse primer: CGCCTGTAAACCTGGATGTCA, SEQ ID NO: 29; hADAMTS13 probe ACCCCTCCAGGAAGA, SEQ ID NO: 30) and a house keeping gene (ACTB, beta Actin). Promoter activity of short liver promoters HCB (Mahler04) and HLP (Mahler05) was clearly confirmed in AAV8.MDTCS (Mahler 04, Mahler05) groups. Average ADAMTS13 plasma levels reached 2-3 U/mL at day 28; increase of expression over time was observed more in AAV8.HLP.Mahler05 treated animals. Mice treated with Mahler01 vector (AAV8.CMV.MDTCS) showed variable expression of plasma ADAMTS13 at day 14 (ranging from 0.6 - 4.6 U/mL) but expression dropped down to background levels in 4 out of 5 mice at day 28. Expression of full length ADAMTS13 driven by HLP (Amadeus 11) was hardly measurable above plasma background levels. Similar results were obtained in the qPCR analyses of liver tissue indicating low ct values (cycle threshold, corresponding to high expression of transgene transcript) in groups Mahler 04 and Mahler 05 and low expression of transgene transcript in group Amadeus 11 (high ct values).

### Example 2: Experimental evidence for the benefit of codon-optimizations of human full length ADAMTS13 for expression by human liver cells (Huh-7)

Codon optimized DNA sequences encoding human full length ADAMTS13 were cloned in mammalian expression plasmids under the control of a constitutive promoter (CMV) and transfected in human hepatoma Huh-7 cells. After 72 hr the cell culture supernatants were collected and expression of secreted ADAMTS13 protein was assessed by ELISA (antigen) or activity assay using FRETS-VWF73 substrate (Fluorescence-Quenching Substrate based on the 73 amino acid peptide of the von Willebrand Factor polypeptide containing the cleavage site for the VWF cleaving protease ADAMTS13). Full length ADAMTS13 (Amadeus 01(A01)) and five different codon optimized variants Strauss (ST) 03, ST04, ST06, ST08, ST13 were tested.

All ST variants resulted in significant increases in antigen production/secretion over unmodified ADAMTS13 sequence (A01) and did not affect the specific activity of ADAMTS13 (Fig. 3).

The Strauss variants were synthetically designed via codon optimization to improve expression in human liver tissue, while reducing immuno-stimulatory CpG motifs to suit gene therapy applications. The optimization was done by using the COOL algorithm (Yu K et al. 2017, Methods Mol Biol. 1472:13-34) which combines individual codon usage (frequency of codon usage within protein coding sequences) with codon context (consecutive pairing of codons within coding sequences). Additional design constraints were added on exclusion sequences from restriction sites, splice sites, and repeats, as well as on a CpG dinucleotide count limit of 5 and GC content (61% +/- 3% or 54% +/- 3%) for the coding sequence.

The resulting scores and characteristics of the designed coding sequences are as follows:

| **Sequence** | **ICU score** | **CC score** | **%CG** | **CpG** | **Design specification** |
|---|---|---|---|---|---|
| **WT** | -0.12599 | -0.11844 | 65.13 | 208 | Wild type sequence |
| **ST03** | -0.10918 | -0.10253 | 58.01 | 5 | CC design, GC content 61 +/-3% |
| **ST04** | -0.07314 | -0.1053 | 58.01 | 5 | ICU-CC design, GC content 61 +/-3% |
| **ST06** | -0.10065 | -0.10074 | 57.05 | 5 | CC design, GC content 54 +/-3% |
| **ST08** | -0.06483 | -0.10528 | 55.53 | 5 | ICU-CC design, GC content 54 +/-3% |
| **ST13** | -0.08742 | -0.10855 | 60.01 | 5 | ICU-CC design, GC content 61 +/-3% |

### Example 3: Experimental evidence for the benefit of the specific internal deletion of thrombospondin-like domains T3,T4,T5, and T6 (delT3-6) to increase the expression/secreted activity overfull length ADAMTS13 by human liver cells (HepG2), but not by other deletion variants.

HepG2 cells were transfected with mammalian expression plasmids expressing ADAMTS13 MDTCS (Mahler01), full length ADAMTS13 (Amadeus01) or specific internal deletion variants (Schubert01, S02, S03) under the control of a constitutive promoter (CMV). Cell culture supernatants were harvested and the ADAMTS13 activity was assessed by using FRETS-VWF73 substrate (Fluorescence-Quenching Substrate based on the 73 amino acid peptide of the von Willebrand Factor polypeptide containing the cleavage site for the VWF cleaving protease ADAMTS13). CMV promoter/enhancer driven expression cassettes harboring full length ADAMTS13 (A13; 5953 bp) and deletion variants Schubert01, in which the thrombospondin domains 3, 4, 5, and 6 are deleted (deIT3-6; 5147 bp), Schubert02, in which the CUB2 domain is deleted (delCUB2; 5552 bp) or Schubert03, in which both the thrombospondin domains 3, 4, 5, and 6 and the CUB2 domain are deleted (delT3-6-delCUB2; 4757 bp), were compared with Mahler01 (CMV-MDTCS; 3716 bp). Figure 4 shows that the internal deletion variant deIT3-6 provided higher activity of ADAMTS13 than full length ADAMTS13 or a delCUB2 or deIT3-6-deICUB2 deletion variant. Based on this data the Schubert01 transgene (delT3-6) was selected for preBruckner01 vector cassette design.

### Example 4: Experimental evidence for the preserved auto-inhibition of ADAMTS13 enzymatic activity (pH-dependent regulation) by vectorized deIT3-6 in A13KO mouse plasma, recapitulating observations in Normal Human Plasma (NHP).

ADAMTS13 activity in individual plasma samples from ADAMSTS13 KO mice (B6.129-Adamts13tm1Dgi/J mouse model, Jackson Labs #007235) treated with either AAV-MDTCS 5 × 10¹² vg/kg (vector genomes per kg, Mahler05); or AAV-delT3-6 5 × 10¹² vg/kg (PreBruckner01) were analyzed under two different assay pH conditions (pH 6.0 vs. pH 7.4). ADAMTS13 activity as a function of pH was assayed using the FRETS-VWF73 assay, using a buffer that included 20 mM Bis-Tris, 20 mM Hepes, and 20 mM Tris.HCl at pH values of pH 6 and pH 7.4 (Assay according to Muia et al. 2014, PNAS Dec 30;111(52):18584-9). Autoinhibition of ADAMTS13 at physiological pH7.4 is evident in samples treated with AAVdelT3-6 as well as in a control using NHP (normal human plasma) as wild-type ADAMTS13 source plasma. The fold increase in activity by shifting to pH 6.0 (i.e. release of auto-inhibition) was calculated by dividing U/ml at pH6 by U/ml at pH7 and is similar in AAVdeIT3-6 treated samples (2.6; 2.1; 2.3; and 2.2-fold) and in NHP samples (1.9 and 2.0 fold) As shown in Figure 5, plasma treated with AAV-MDTCS variant is fully active at pH7.4 and the shift to pH 6.0 does not induce an increase in activity.

### Example 5: Experimental evidence for the potency and durability of liver directed expression of ADAMTS13 activity mediated by transduction with AAVdelT3-6

AAVdeIT3-6 (PreBruckner01; HLP promoter directed expression of ADAMTS13 deIT3-6, administered at 5 × 10¹³ vg/kg or 5 × 10¹² vg/kg), or AAV vectors encoding a MDTCS variant (either expressed by liver specific promoter HLP (Mahler05) or a constitutive promoter (Mahler01), administered at 5 × 10¹² vg/kg) were administered intravenously to male ADAMTS13 KO mice (B6.129-Adamts13tm1Dgi/J mouse model, Jackson Labs #007235), and plasma samples were collected (retroorbital bleeds) 2, 4, 6, 9, 12, 16 and 20 weeks after dosing. ADAMTS13 activity was determined in plasma samples using FRETS-VWF73 substrate (Fluorescence-Quenching Substrate based on the 73 amino acid von Willebrand Factor polypeptide for the VWF cleaving protease ADAMTS13). Age range at dosing: 16 - 18 weeks; Age range at pharmacologic challenge: 22 - 24 weeks; Age range at study end week 24: 41 - 42 weeks.

As shown in Figure 6, liver directed expression of ADAMTS13 deIT3-6 provided stable ADAMTS13 plasma activity over the whole time period, which was significantly higher than activity of the MDTCS variant.

### Example 6: Disease-modification by liver specific lead vector in challenged ADAMTS13 KO mice

The mouse model of human cTTP encompasses ADAMTS13 deficient mice (B6.129-Adamts13tm1Dgi/J mouse model, Jackson Labs #007235) challenged by an intravenous injection of the only known substrate (high molecular weight VWF multimers) triggering the latent thrombotic disease phenotype (micro-thrombi formation in peripheral sites) reflected by low platelet counts in blood test (thrombocytopenia).

Mice were administered buffer, an irrelevant ineffective vector (Mahler01), AAV-MDTCS (5 × 10¹² vg/kg, Mahler05); AAV-delT3-6 (5 × 10¹² vg/kg, PreBruckner01) or AAV-deIT3-6 at a higher concentration (5 × 10¹³ vg/kg, PreBruckner01). 3-4 weeks after treatments the number of platelets was determined (pre-challenge) and again 24 hours after challenge with recombinant VWF (post-challenge). Pharmacologic challenge of AOAMTS13KO mice induced clear clinical signs of TTP and microthrombi formation (reduced platelet counts) in the groups treated with buffer or an irrelevant ineffective vector. Pretreatment with PreBruckner01 at 5 × 10¹² vg/kg was fully protective in most animals, while pretreatment with PreBruckner01 at 5 × 10¹³ vg/kg fully protected all animals from clinical signs of TTP and thrombocytopenia (see Figure 7).

Pharmacologic challenge: 4 weeks post transductions. Age range at dosing: 16 - 18 weeks; Age range at pharmacologic challenge: 22 - 24 weeks; Age range at study end week 24: 41 - 42 weeks.

### Example 7: Minimum efficacious dose finding and pharmacological effective dose estimation in the ADAMTS13 KO mouse

AAVdelT3-6 (PreBruckner01) was administered intravenously to male ADAMTS13 KO mice at five different doses, 1 × 10¹³, 3 × 10¹², 1 × 10¹², 3 × 10¹¹, and 1 × 10¹¹ vg/kg. Plasma samples were collected (retroorbital bleeds) 2, 4 and 8 weeks after dosing. ADAMTS13 activity was determined in plasma samples using FRETS-VWF73 substrate (Fluorescence-Quenching Substrate based on the 73 amino acid von Willebrand Factor polypeptide for the VWF cleaving protease ADAMTS13). ADAMTS13 activity is fully restored in mouse plasma at all dose levels ≥ 3 × 10¹¹ vg/kg, at all timepoints tested, considering the reference level in WT mouse of 0.4 U/mL (see Figure 2A, pre-administration). A large titratable pharmacodynamic range over the entire dose range tested is evident. The data suggest that a relatively low and clinically effective starting dose can be estimated. The targeted plasma activity level restoring to 50-150% of normal activity in human is 0.5 U/mL to 1.5 U/mL. An activity level of less than 10% confirms the diagnosis of TTP in patients with evidence of hemolysis and thrombocytopenia. (Stanley et al., 2024 StatPearls, PMID: 28613472).

A reasonable clinically feasible starting dose correcting ADAMTS13 levels to at least 10% of normal levels in human patients could include a dose of 3 × 10¹² vg/kg. A clinical dose correcting ADAMTS13 levels to >50% of normal levels could include 9 × 10¹² vg/kg.

### Example 8: Expression cassette optimization - Expression by human liver cell line can be effectively improved by addition of regulatory elements inserted 5'and 3'of the ADAMTS13 deIT3-6 transgene.

Human liver cell line Huh-7 was transfected with expression cassettes encoding ADAMTS13 deIT3-6 under the control of a liver specific promoter (HLP_A13deIT3-6 = Bruckner01); the HLP promoter and a chimeric beta-globin intron (SEQ ID NO: 6) inserted 5` of the transgene (HLP_chimeric β-globin intron_A13delT3-6); the HLP promoter and Woodchuck Hepatitis Virus Posttranscriptional Regulatory Element (SEQ ID NO: 7) inserted 3` of the transgene (HLP_A13deIT3-6_WPRE); or the HLP promoter and a chimeric beta-globin intron inserted 5' of the transgene and Woodchuck Hepatitis Virus Posttranscriptional Regulatory Element inserted 3` of the transgene (HLP_chimeric β-globin intron_A13deIT3-6_WPRE). Cell culture supernatants were harvested and concentrated 9-fold and ADAMTS13 activity was determined using FRETS-VWF73 substrate (Fluorescence-Quenching Substrate based on the 73mer von Willebrand Factor polypeptide for the VWF cleaving protease ADAMTS13). All three tested variants robustly increased the expression (activity in supernatants) of ADAMTS13 delT3- 6 compared to the parent cassette HLP_A13deIT3-6 (Bruckner01) which does not contain additional regulatory elements (see Figure 9).

## Claims

1. A gene therapy plasmid comprising a recombinant gene expression cassette, comprising a nucleic acid sequence encoding a variant of ADAMTS13, wherein the nucleic acid sequence encoding a variant of ADAMTS13 comprises one proximal thrombospondin domain and at least one distal thrombospondin domains of ADAMTS13; wherein the gene therapy plasmid is suitable for viral delivery.

2. The gene therapy plasmid of claim 1, wherein the nucleic acid sequence encoding a variant of ADAMTS13 comprises a nucleic acid sequence encoding thrombospondin-1 (T1) domain and a nucleic acid sequence encoding thrombospondin-8 (T8) domain of ADAMTS13, preferably wherein the nucleic acid sequence encoding a variant of AOAMTS 13 further comprises a nucleic acid sequence encoding thrombospondin-7 (T7) domain of ADAMTS13.

3. The gene therapy plasmid of any one of claims 1 or 2, wherein the nucleic acid sequence encoding a variant of ADAMTS13 further comprises a nucleic acid sequence encoding thrombospondin-2 (T2) domain of ADAMTS13.

4. The gene therapy plasmid of any one of the preceding claims, wherein the nucleic acid sequence encoding a variant of ADAMTS13 further comprises a nucleic acid sequence encoding at least one CUB (complement components C1r and C1s, sea urchin protein Uegf, and bone morphogenetic protein-1) domain of ADAMTS13, preferably, wherein the nucleic acid sequence encoding a variant of ADAMTS13 further comprises a nucleic acid sequence encoding metalloprotease (M), disintegrin-like (D), Cys-rich (C) and spacer (S) domains of ADAMTS13.

5. The gene therapy plasmid of any one of the preceding claims, wherein the nucleic acid sequence encoding a variant of ADAMTS13 encodes a ADAMTS13 variant with deletion of thrombospondin-3, -4, -5, and -6 domains (delT3-T6) of ADAMTS13.

6. The gene therapy plasmid of any one of the preceding claims, wherein the recombinant gene expression cassette
(i) is flanked by viral ITR sequences;
(ii) comprises a viral origin of replication (ORI);
(iii) further comprises a tissue-specific promoter operatively linked to the nucleic acid sequence encoding a variant of ADAMTS13;
(iv) further comprises a nucleic acid sequence comprising one or more 5' intron sequences; and/or
(v) further comprises a nucleic acid sequence comprising one or more Woodchuck Hepatitis Virus posttranscriptional regulatory element (WPRE) sequences.

7. The gene therapy plasmid according to any one of the preceding claims, wherein the tissue-specific promoter is a liver-specific promoter, preferably the liver-specific promoter is selected from the group consisting of HLP, hAAT, Alb, HBV, HCB, LP1, LP1b, and TTR; more preferably the liver-specific promoter is selected from the group consisting of HLP and HCB.

8. The gene therapy plasmid according to any one of the preceding claims, wherein the nucleic acid sequence encoding a variant of ADAMTS13 comprises a nucleic acid sequence that is at least 70% identical to the nucleic acid sequence set forth in SEQ ID NO: 1.

9. The gene therapy plasmid according to any one of the preceding claims, wherein the variant of ADAMTS13 comprises a nucleic acid sequence encoding at least one CUB domain and is capable of autoinhibition of the metalloprotease.

10. An isolated polynucleotide comprising the gene therapy plasmid of any one of claims 1 to 9 or the recombinant gene expression cassette as defined in any one of claims 1 to 9.

11. A recombinant virus comprising the recombinant gene expression cassette as defined in any one of claims 1 to 9 or the isolated polynucleotide as defined in claim 10, preferably wherein the recombinant virus is from the genus *Dependoparovirus,* more preferably wherein the recombinant virus is Adeno-associated virus (AAV).

12. A host cell comprising the gene therapy plasmid of any one of claims 1 to 9, the isolated polynucleotide of claims 10, or the recombinant virus of claim 11.

13. A pharmaceutical composition comprising the gene therapy plasmid of any one of claims 1 to 9, the isolated polynucleotide of claims 10, the recombinant virus of claim 11, or the host cell of claim 12, and one or more pharmaceutically acceptable excipients.

14. The gene therapy plasmid of any one of claims 1 to 9, the isolated polynucleotide of claims 10, the recombinant virus of claim 11, the host cell of claim 12, or the pharmaceutical composition of claim 13 for use as a medicament.

15. The gene therapy plasmid of any one of claims 1 to 9, the isolated polynucleotide of claims 10, the recombinant virus of claim 11, the host cell of claim 12, or the pharmaceutical composition of claim 13 for use in treatment of Congenital Thrombotic Thrombocytopenic Purpura (cTTP).

16. The gene therapy plasmid, the isolated polynucleotide, the recombinant virus, the host cell, or the pharmaceutical composition for use according to claim 15, wherein the treatment
(i) restores plasma activity level of ADAMTS13 to at least 10% of normal activity, preferably to at least 50% of normal activity; when measured with an activity assay using a von Willebrand Factor Fluorescence-Quenching Substrate; or
(ii) restores plasma activity level of ADAMTS13 to 50% to 150% of normal activity; when measured with an activity assay using a von Willebrand Factor Fluorescence-Quenching Substrate.
optionally wherein the restored plasma activity level of ADAMTS13 remains stable over at least 3 months.

17. The recombinant virus for use according to any one of claims 14 to 16, wherein the recombinant virus is administered at a dose between 3 × 10¹² vg/kg to 9 ×10¹² vg/kg.
